# EUROPÄISCHE PATENTSCHRIFT

(11) **EP 0 545 099 B1**
(45) Veröffentlichungstag und Bekanntmachung des Hinweises auf die Patenterteilung: **05.03.1997**
(21) Anmeldenummer: 92119105.2
(22) Anmeldetag: 07.11.1992
(51) Int. Cl.: C07D 213/82, C07D 231/14, C07D 277/56, C07D 263/34, C07D 307/68, C07D 309/28, C07D 327/06, C07C 233/64, A01N 37/22, A01N 43/00

(54) **Säureanilid-Derivate und ihre Verwendung zur Bekämpfung von Botrytis**
Anilide derivatives and their use to combat Botrytis
Dérivés d'anilide et leur utilisation pour combattre Botrytis

(30) Priorität: 22.11.1991 DE 4138387; 18.02.1992 DE 4204764; 18.02.1992 DE 4204766; 18.02.1992 DE 4204767; 18.02.1992 DE 4204768
(43) Veröffentlichungstag der Anmeldung: 09.06.1993
(73) Patentinhaber: BASF Aktiengesellschaft, 67063 Ludwigshafen (DE)
(72) Erfinder: Eicken, Karl, Dr., W-6706 Wachenheim (DE); Goetz, Norbert, Dr., W-6520 Worms 1 (DE); Harreus, Albrecht, Dr., W-6700 Ludwigshafen (DE); Ammermann, Eberhard, Dr., W-6148 Heppenheim (DE); Lorenz, Gisela, Dr., W-6730 Neustadt (DE); Rang, Harald, Dr., W-6700 Ludwigshafen (DE)

(56) Entgegenhaltungen:
- EP-A- 86 111
- EP-A- 480 258
- EP-A- 0 256 503
- EP-A- 0 276 177
- EP-A- 0 279 239
- EP-A- 0 296 673
- EP-A- 0 314 428
- EP-A- 0 371 950
- WO-A-91/01311
- WO-A-93/11117
- DE-A- 1 914 954
- DE-A- 2 409 011
- DE-A- 2 417 216
- DE-A- 2 434 430
- DE-A- 2 611 601
- DE-A- 2 635 818
- FR-A- 1 546 183
- FR-A- 2 337 997
- JP-A-53 072 823
- JP-B-54 038 109
- PHYTOPATHOLOGY Bd. 57, Nr. 11, 1967, ST. PAUL Seiten 1256 - 1257 L.V. EDGINGTON ET AL. 'Fungitoxic spectrum of oxathiin compounds.'
- CHEMICAL ABSTRACTS, vol. 117, no. 23, 7. Dezember 1992, Columbus, Ohio, US; abstract no. 228322b, M. ODA ET AL. 'Structure-activity relations of 2-chloropyridine 3-carboxamide fungicides.' Seite 303 ;
- CHEMICAL ABSTRACTS, vol. 81, no. 19, 11. November 1974, Columbus, Ohio, US; abstract no. 115750, M.F.A. ABDEL-LATEEF ET AL. 'Systemic and chemotherapeutic fungicidal activity-chemical structure relation of some 4-methyl-5-thiazolecarboxylic acid derivatives. Laboratory screening tests.' Seite 142 ;
- Pestic.Biochem.Physiol.31(2),129-145(1988)
- ca 59 : 5945f
- Pestic.Biochem.Physiol.34(3),255-276(1989)
- Pestic.Biochem.Physiol.27(3),249-260(1987)

## Beschreibung

Die vorliegende Erfindung betrifft die Verwendung von Carbonsäureamiden der allgemeinen Formel I 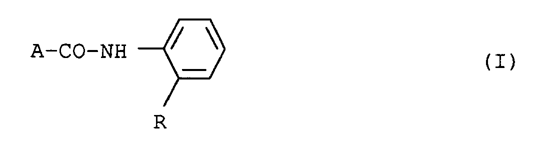 in der die Reste die folgenden Bedeutungen haben:
- A:
- A1:: Pyridin-3-yl, substituiert in 2-Stellung durch Halogen, Methyl, Trifluormethyl, Methoxy, Methylthio, Methylsulfinyl, Methylsulfonyl,
- A2:: Thiazol-5-yl, substituiert in 2- und 4-Stellung durch Wasserstoff, Methyl, Trifluormethyl,
- A3:: Thiazol-4-yl, substituiert in 2- und 5-Stellung durch Wasserstoff, Methyl, Chlor, Trifluormethyl,
- A4:: 1-Methylpyrazol-4-yl, substituiert in 3-Stellung durch Methyl, Chlor, Trifluormethyl und in 5-Stellung durch Chlor, oder
- A5:: Oxazol-5-yl, substituiert in 2- und 4-Stellung durch Wasserstoff, Methyl, Chlor und
- R: gegebenenfalls durch Halogen substituiertes C₂-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy,
gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy, gegebenenfalls durch C₁-C₄-Alkyl, C₁-C₄-Alkoxy, C₁-C₄-Alkylthio, Halogen, substituiertes Phenyl,
ausgenommen die Verbindungen I, in denen
a)
   - A =: A1, welches in 2-Stellung Halogen trägt,
   - R =: gegebenenfalls durch Halogen substituiertes Ethyl oder gegebenenfalls durch Halogen substituiertes Ethoxy
b)
   - A =: A1, welches in 2-Stellung Halogen trägt,
   - R =: unsubstituiertes Phenyl
c)
   - A =: A2,
   - R =: gegebenenfalls durch Halogen substituiertes Ethyl und gegebenenfalls durch Halogen substituiertes Ethoxy
d)
   - A =: A2,
   - R =: unsubstituiertes Phenyl
e)
   - A =: A4,
   - R =: gegebenenfalls substituiertes: Cycloalkyl, Cycloalkenyl, Cycloalkyloxy oder Cycloalkenyloxy und
f)
   - A =: A4,
   - R =: gegebenenfalls durch Halogen substituiertes Ethyl,
zur Bekämpfung von Botrytis.

Ferner betrifft die vorliegende Erfindung neue Carbonsäureamide I sowie diese enthaltende Mittel.

Es ist bekannt, Nicotinsäureanilide, z.B. das 2-Chlornicotinsäure-2'-ethylanilid (US-A 4 001 416) oder das 2-Chlornicotinsäure-3'-isopropylanilid (DE-A 26 11 601) als Fungizide zu verwenden.

Ferner ist allgemein bekannt, daß bestimmte 2-Chlor-4-trifluormethylthiazol-5-carbonsäureamide eine fungizide Wirkung zeigen (vgl. EP-A 279 239).

Es wurde nun gefunden, daß die eingangs definierten Carbonsäureamide eine gute Wirkung gegen Botrytis besitzen.

Im Hinblick auf ihre Wirksamkeit sind Verbindungen bevorzugt, in denen die Substituenten folgende Bedeutung haben:

Halogen z.B. Fluor, Chlor, Brom,
Alkyl wie insbesondere Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl, Dodecyl wobei das Alkyl ein bis drei der vorstehend genannten Halogenatome, insbesondere Fluor und Chlor tragen kann,
Alkenyl, wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl,
wobei das Alkenyl ein bis drei der vorstehend genannten Halogenatome, insbesondere Fluor und Chlor tragen kann,
Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,2-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl,
Alkoxy wie insbesondere Ethoxy, Propoxy, 1-Methylethoxy, Butoxy, 1-Methylpropoxy, 2-Methylpropoxy, 1,1-Dimethylethoxy, n-Pentyloxy, 1-Methylbutoxy, 2-Methylbutoxy, 3-Methylbutoxy, 1,2-Dimethylpropoxy, 1,1-Dimethylpropoxy, 2,2-Dimethylpropoxy, 1-Ethylpropoxy, n-Hexyloxy, 1-Methylpentyloxy, 2-Methylpentyloxy, 3-Methylpentyloxy, 4-Methylpentyloxy, 1,2-Dimethylbutoxy, 1,3-Dimethylbutoxy, 2,3-Dimethylbutoxy, 1,1-Dimethylbutoxy, 2,2-Dimethylbutoxy, 3,3-Dimethylbutoxy, 1,1,2-Trimethylpropoxy, 1,2,2-Trimethylpropoxy, 1-Ethylbutoxy, 2-Ethylbutoxy, 1-Ethyl-2-methylpropoxy, n-Heptyloxy, 1-Methylhexyloxy, 2-Methylhexyloxy, 3-Methylhexyloxy, 4-Methylhexyloxy, 5-Methylhexyloxy, 1-Ethylpentyloxy, 2-Ethylpentyloxy, 1-Propylbutoxy, Octyloxy, Decyloxy, Dodecyloxy, wobei das Alkoxy ein bis drei der vorstehend genannten Halogenatome, insbesondere Fluor und Chlor tragen kann,
Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, insbesondere 2-Propenyloxy, 2-Butenyloxy, 3-Methyl-2-butenyloxy, und 3-Methyl-2-pentenyloxy,
wobei das Alkenyloxy ein bis drei der vorstehend genannte Halogenatome, insbesondere Fluor und Chlor tragen kann,
Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Alkinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-3-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy, 2-Butinyloxy, 1-Methyl-2-propinyloxy und 1-Methyl-2-butinyloxy,
C₃-C₆-Cycloalkyl, wie Cyclopropyl, Cyclobutyl, Cyclopentyl, Cyclohexyl, wobei das Cycloalkyl gegebenenfalls durch einen bis drei C₁-C₄-Alkylreste substituiert ist,
C₄-C₆-Cycloalkenyl, wie Cyclobutenyl, Cyclopentenyl, Cyclohexenyl, das gegebenenfalls durch einen bis drei C₁-C₄-Alkylreste substituiert ist,
C₅-C₆-Cycloalkoxy wie Cyclopentyloxy oder Cyclohexyloxy, das durch einen bis drei C₁-C₄-Alkylreste substituiert sein kann,
C₅-C₆-Cycloalkenyloxy wie Cyclopentyloxy oder Cyclohexaryloxy, das durch einen bis drei C₁-C₄-Alkylreste substituiert sein kann,
Bevorzugt ist die Verwendung von Carbonsäureamiden der allgemeinen Formel I 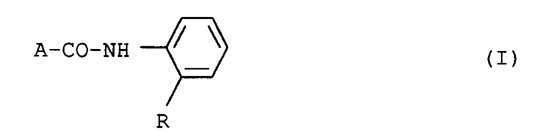 in der die Reste die folgenden Bedeutungen haben:
A 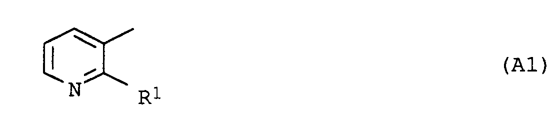
R¹ Halogen, Methyl, Trifluormethyl, Methoxy, Methylthio, Methylsulfinyl, Methylsulfonyl und
R gegebenenfalls durch Halogen substituiertes C₂-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, C₃-C₆-Cycloalkyl, C₄-C₆-Cycloalkenyl, C₅-C₆-Cycloalkyloxy, C₅-C₆-Cycloalkenyloxy, ausgenommen die Verbindungen I, in denen
- A =: A1, welches in 2-Stellung Halogen trägt,
- R =: gegebenenfalls durch Halogen substituiertes Ethyl oder gegebenenfalls durch Halogen substituiertes Ethoxy, zur Bekämpfung von Botrytis.

Die Verbindungen der Formel I, in denen A für den Rest A1 steht, erhält man beispielsweise, in dem man ein entsprechend substituiertes Nicotinsäurehalogenid der Formel 1 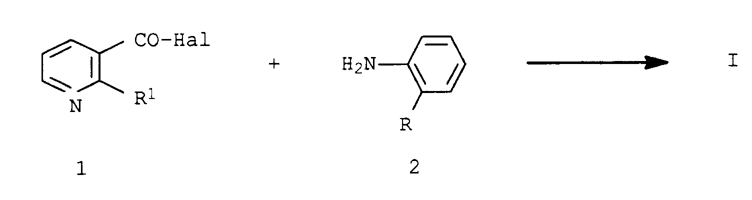 (Hal = Chlor oder Brom) mit einem ortho-substituierten Anilin der Formel 2 in Gegenwart einer Base umsetzt. Die Nicotinsäuren bzw. deren Halogenide der Formel 1 sind bekannt. Die Aniline der Formel 2 sind bekannt oder können nach bekannten Verfahren hergestellt werden (Helv. Chim. Acta 60, 978 (1977); Zh. Org. Khim. 26, 1527(1990); Heterocyclus 26, 1885 (1987); Izv. Akad. Nauk. SSSR, Ser. Khim 1982, 2160).

Insbesondere bevorzugt sind Verbindungen der Formel I, in denen der Rest R¹ für Chlor steht und der Rest R die eingangs erwähnte Bedeutung hat.

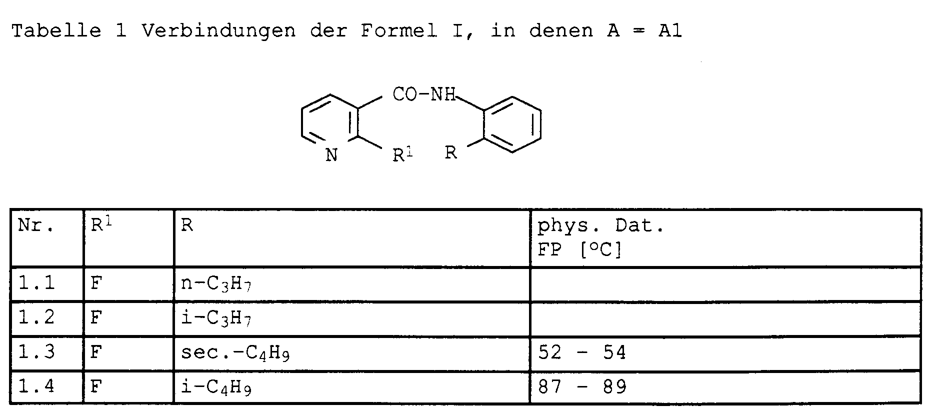

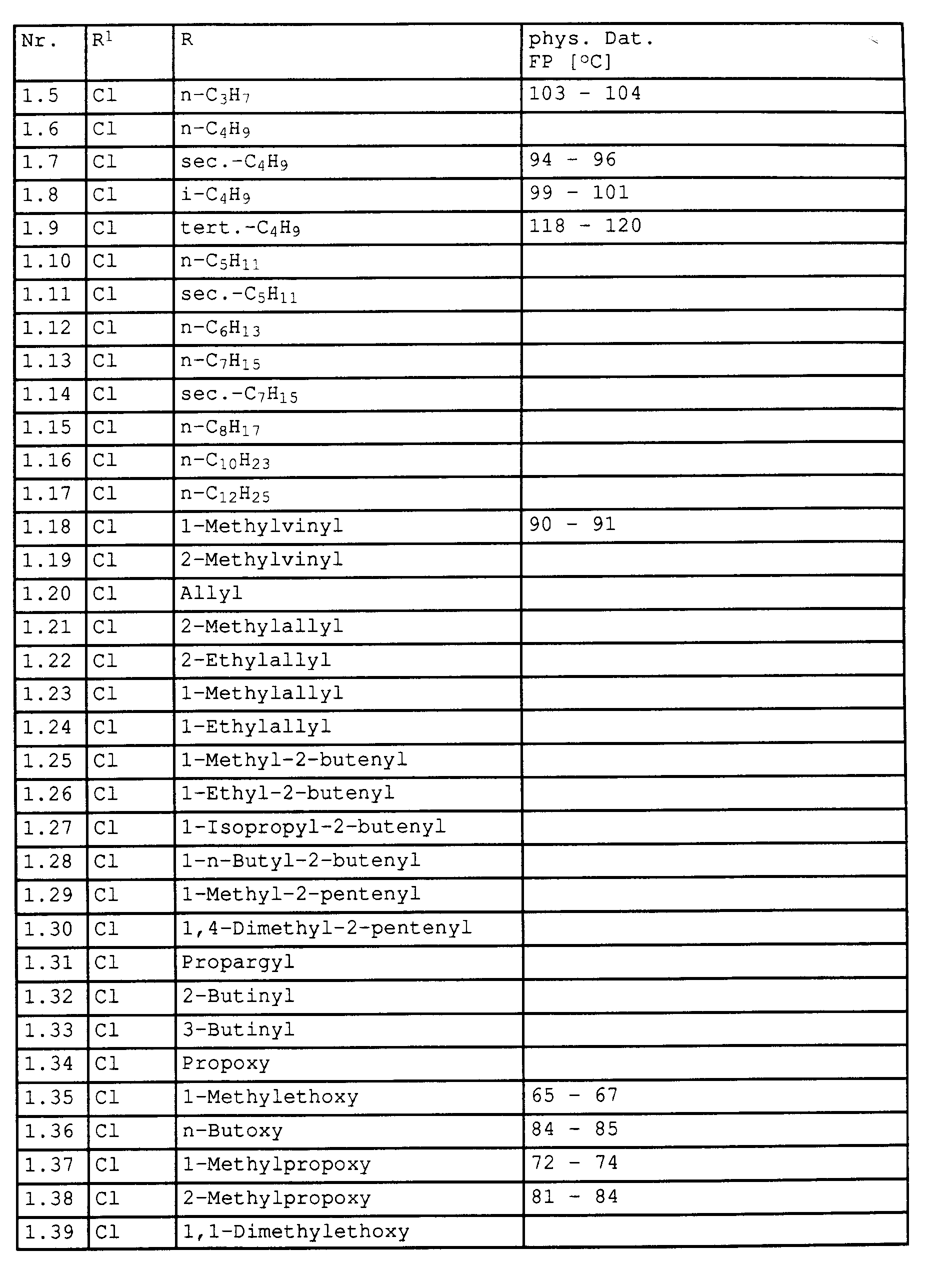

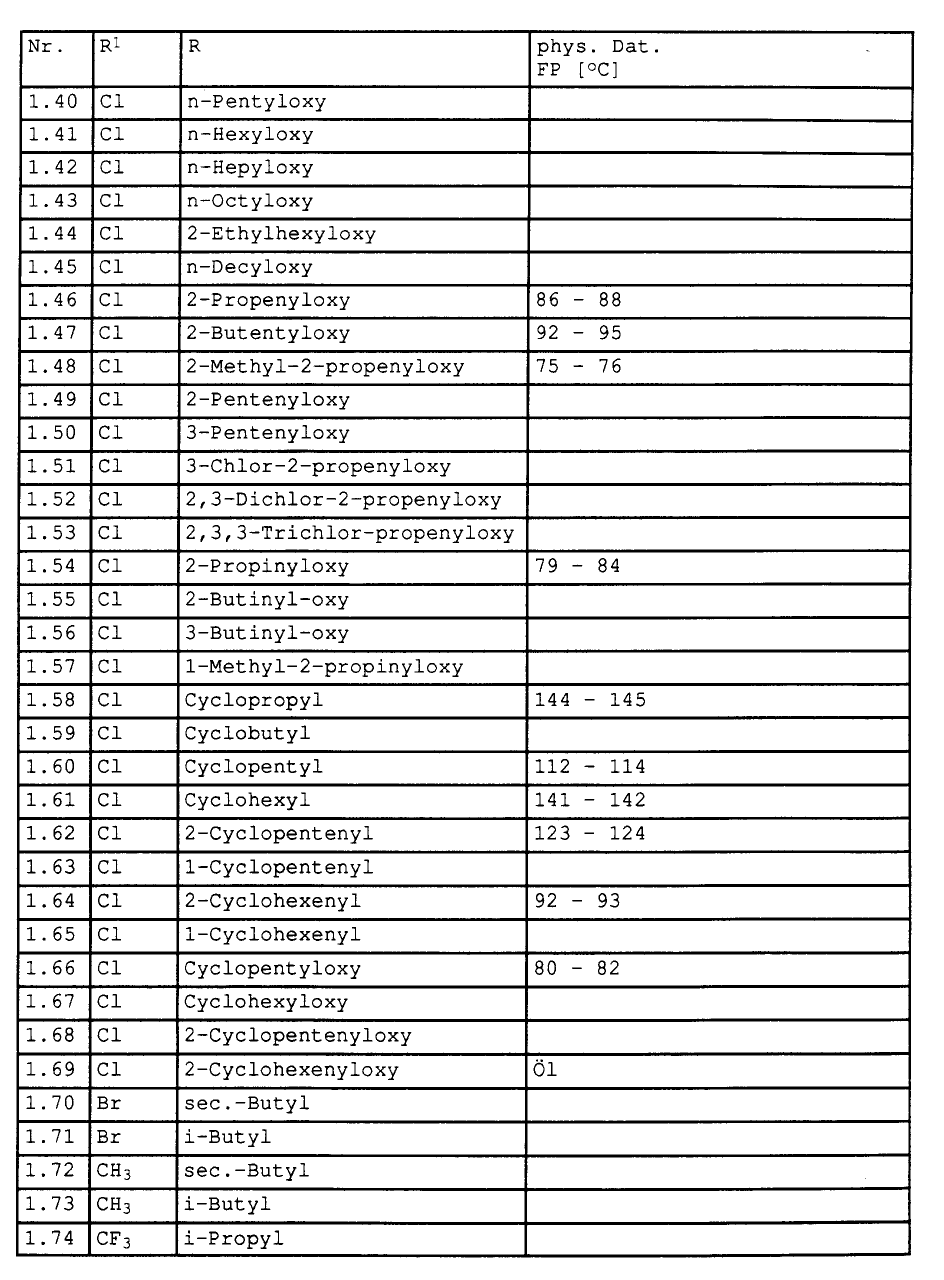

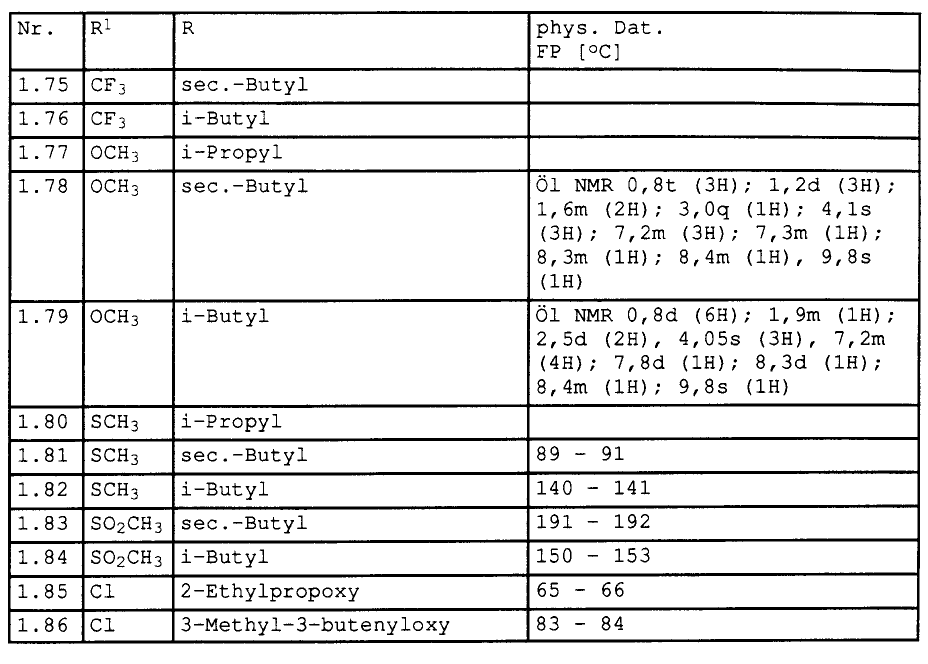

### Herstellungsbeispiele

### Beispiel 1

Zu einer Lösung von 2,7 g 2-n-Propylanilin und 2,0 g Triethylamin in 30 ml Tetrahydrofuran tropft man bei 0°C 3,5 g 2-Chlornicotinsäurechlorid und rührt noch 2 Stdn. bei 0°C. Nach Verdünnen mit 300 ml Wasser isoliert man 3,2 g 2-Chlornicotinsäure-2-n-propylanilid von Fp.: 103 - 104°C (Nr. 1.5).

### Beispiel 2

4,4 g 2-Chlornicotinsäure-2-sec.-butylanilid (Tabelle 1, Nr. 1.7) werden in einer Lösung von 5,5 g 30 % Natriummethylat-Lösung in 20 ml Methanol 2 Stdn. am Rückfluß gekocht. Nach Verdünnen mit 250 ml Wasser wird zweimal mit je 100 ml Essigester extrahiert. Aus den vereinigten organ. Phasen isoliert man nach Trocknen und Verdampfen des Lösungsmittels 3,8 g 2-Methoxi-nicotinsäure-2-sec.-butylanilid als Öl. (Nr. 1.78).

### Beispiel 3

Aus 5,7 g 2-Methylthionicotinsäurechlorid, 4,6 g 2-sec-Butylanilin und 3,1 g Triethylamin erhält man analog zu Beispiel 1 6,6 g 2-Methylthionicotinsäure-2-sec.-butylanilid vom Fp.: 89 - 91°C (Nr. 1.81).

### Beispiel 4

In eine Mischung aus 2,00 g des obigen Produkts (Beispiel 3) in 5 ml Eisessig und 0,13 g Natriumwolframat tropft man unter Rühren bei 35°C 2,20 g 30 % Wasserstoffperoxid zu und rührt 3 Stdn. bei 35°C nach. Nach Verdünnen mit 15 ml Wasser, Absaugen der Kristalle, Waschen mit Wasser und Trocknen erhält man 1,7 g 2-Methylsulfonylnicotinsäure-2-sec.-butylanilid vom FP.: 191 - 192°C (Nr. 1.83).

Neu sind die Carbonsäureamide der allgemeinen Formel I, in der die Reste die folgenden Bedeutungen haben:
A 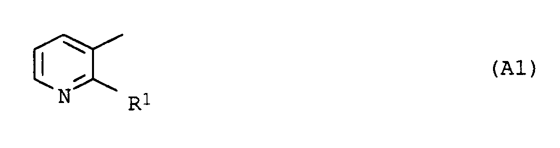 worin
- R¹: Halogen, Methyl, Trifluormethyl, Methoxy, Methylsulfinyl, Methylsulfonyl,
- R: gegebenenfalls durch Halogen substituiertes C₃-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, C₃-C₆-Cycloalkyl, C₄-C₆-Cycloalkenyl, C₅-C₆-Cycloalkyloxy, C₅-C₆-Cycloalkenyloxy,
ausgenommen die Verbindungen I, in denen
- A =: A1, welches in 2-Stellung Chlor trägt,
- R =: iso-Propyl und
- A =: A1, welches in 2-Stellung Halogen trägt,
- R =: gegebenenfalls durch Halogen substituiertes Ethoxy.

Weiterhin bevorzugt ist die Verwendung von Carbonsäureamiden der allgemeinen Formel I 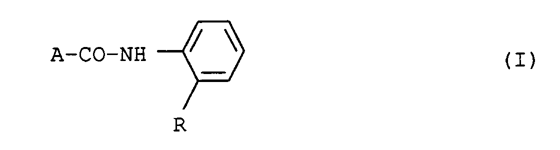 in der die Reste die folgenden Bedeutungen haben:
- A: 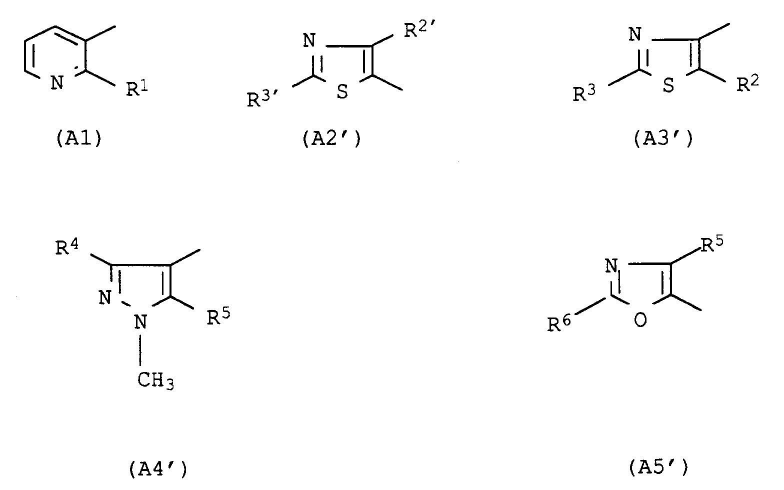
worin die Substituenten R¹ bis R⁶ ihrerseits bedeuten:
R¹ Trifluormethyl, Chlor,
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;

- R: Phenyl, welches einfach substituiert sein kann durch C₁-C₄-Alkyl, C₁-C₄-Alkoxy, C₁-C₄-Alkylthio, Halogen,
ausgenommen die Verbindungen I, in denen
a)
   - A =: A1, welches in 2-Stellung Halogen trägt,
   - R =: unsubstituiertes Phenyl und
b)
   - A =: A2',
   - R =: unsubstituiertes Phenyl,
zur Bekämpfung von Botrytis.

Diese Verbindungen erhält man beispielsweise, indem man ein entsprechend substituiertes Carbonsäurehalogenid der Formel 3

A ― CO ― Hal 3

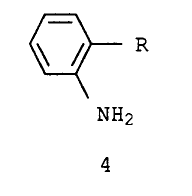

(Hal = Chlor oder Brom) mit einem ortho-substituierten Anilin der Formel 4 in Gegenwart einer Base umsetzt. Die Carbonsäuren bzw. deren Halogenide der Formel 3 sind bekannt. Die Aniline der Formel 4 sind z. Teil bekannt oder können nach bekannten Verfahren hergestellt werden (Tetrahedron Letters, Vol. 28, S. 5093 (1987); THL Vol. 29, 5463 (1988)).

**Tabelle 2**

| Nr. | A | R¹ | R^{2'} | R^{3'} | R⁴ | R⁵ | R | phys. Daten [°C] |
|---|---|---|---|---|---|---|---|---|
| 2.1 | A1 | Cl | - | - | - | - | 2-F-Phenyl | |
| 2.2 | A1 | Cl | - | - | - | - | 2-CH₃-Phenyl | 71 - 73 |
| 2.3 | A1 | Cl | - | - | - | - | 2-Cl-Phenyl | |
| 2.4 | A1 | Cl | - | - | - | - | 2-OCH₃-Phenyl | |
| 2.5 | A1 | Cl | - | - | - | - | 3-F-Phenyl | |
| 2.6 | A1 | Cl | - | - | - | - | 3-Cl-Phenyl | 95 - 98 |
| 2.7 | A1 | Cl | - | - | - | - | 3-CH₃-Phenyl | |
| 2.8 | A1 | Cl | - | - | - | - | 3-OCH₃-Phenyl | |
| 2.9 | A1 | Cl | - | - | - | - | 3-OiC₃H₇-Phenyl | |
| 2.10 | A1 | Cl | - | - | - | - | 3-Br-Phenyl | |
| 2.11 | A1 | Cl | - | - | - | - | 4-F-Phenyl | 156 - 157 |
| 2.12 | A1 | Cl | - | - | - | - | 4-Cl-Phenyl | |
| 2.13 | A1 | Cl | - | - | - | - | 4-CH₃-Phenyl | |
| 2.14 | A1 | Cl | - | - | - | - | 4-OCH₃-Phenyl | |
| 2.15 | A1 | Cl | - | - | - | - | 4-SCH₃-Phenyl | |
| 2.16 | A2' | - | CF₃ | CH₃ | - | - | 2-F-Phenyl | |
| 2.17 | A2' | - | CF₃ | CH₃ | - | - | 3-F-Phenyl | |
| 2.18 | A2' | - | CF₃ | CH₃ | - | - | 4-F-Phenyl | |
| 2.19 | A4' | - | - | - | CH₃ | Cl | 2-F-Phenyl | |
| 2.20 | A4' | - | - | - | CH₃ | Cl | 3-F-Phenyl | |
| 2.21 | A4' | - | - | - | CH₃ | Cl | 4-F-Phenyl | |
| 2.22 | A4' | - | - | - | CF₃ | Cl | 2-F-Phenyl | |
| 2.23 | A4' | - | - | - | CF₃ | Cl | 4-F-Phenyl | |
| 2.24 | A2' | - | CH₃ | CH₃ | - | - | Phenyl | 136-137 |
| 2.25 | A1 | CF₃ | - | - | - | - | Phenyl | |
| 2.26 | A2' | - | CF₃ | CH₃ | - | - | Phenyl | 116-118 |
| 2.27 | A3' | - | CH₃* | CH₃** | - | - | Phenyl | |
| 2.28 | A3' | - | Cl* | Cl** | - | - | Phenyl | |
| 2.29 | A4' | - | - | - | CH₃ | Cl | Phenyl | 108-109 |
| 2.30 | A4' | - | - | - | CF₃ | Cl | Phenyl | |
| 2.31 | A4' | - | - | - | CH₃ | CH₃ | Phenyl | |

| | | | | | | | | |
|---|---|---|---|---|---|---|---|---|
| * R² | | | | | | | | |
| ** R³ | | | | | | | | |

Neu sind die Carbonsäureamide der allgemeinen Formel I, in der die Reste die folgenden Bedeutungen haben:
- A: 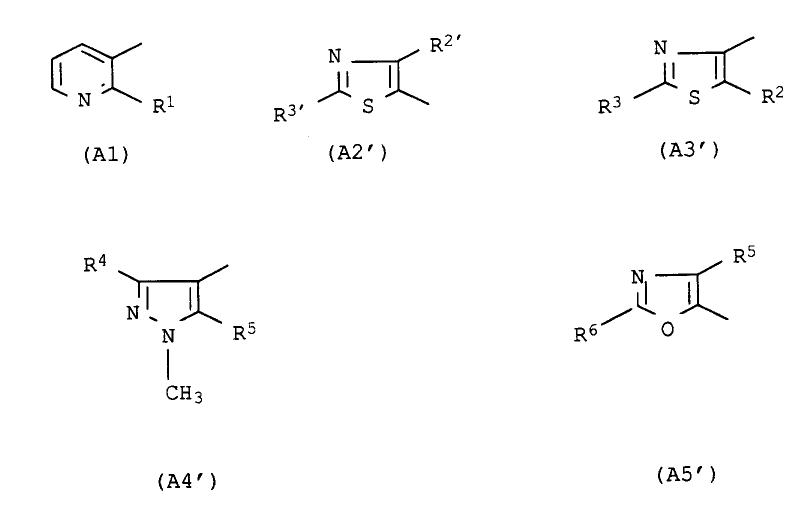
worin die Substituenten R¹ bis R⁶ ihrerseits bedeuten:
R¹ Trifluormethyl, Chlor,
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;

- R: Phenyl, welches einfach substituiert sein kann durch C₁-C₄-Alkyl, C₁-C₄-Alkoxy, C₁-C₄-Alkylthio, Halogen,
ausgenommen die Verbindungen I, in denen
a)
   - A =: A1, welches in 2-Stellung Halogen trägt,
   - R =: unsubstituiertes Phenyl,
b)
   - A =: A2',
   - R =: unsubstituiertes Phenyl und
c)
   - A =: A4',
   - R =: unsubstituiertes Phenyl.

Bevozugt ist ferner die Verwendung von Carbonsäureamiden der allgemeinen Formel I 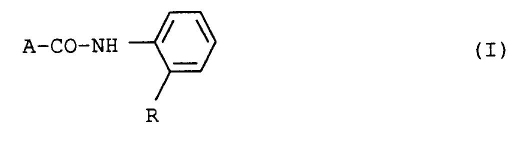 in der die Reste die folgenden Bedeutungen haben:
- A: 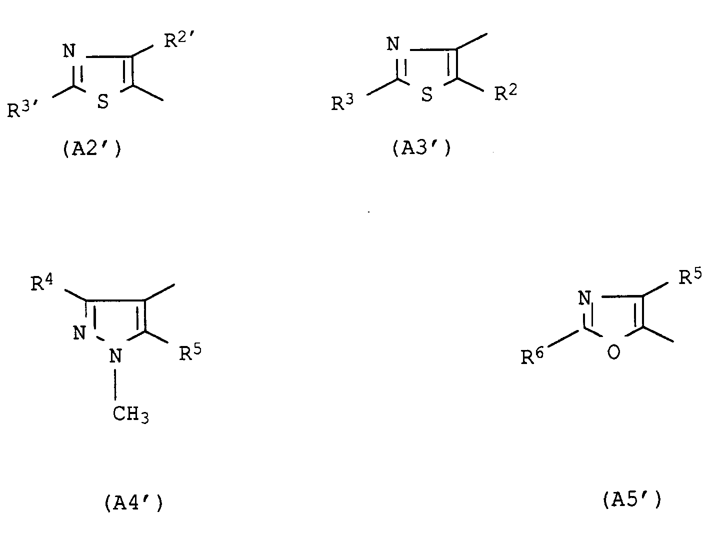
worin die Substituenten R² bis R⁶ ihrerseits bedeuten:
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;

- R: gegebenenfalls durch Halogen substituiertes C₃-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy,
ausgenommen die Verbindungen I, in denen
a)
   - A =: A2',
   - R =: gegebenenfalls durch Halogen substituiertes Ethoxy und
b)
   - A =: A4',
   - R =: gegebenenfalls substituiertes: Cycloalkyl, Cycloalkenyl, Cycloalkyloxy oder Cycloalkenyloxy,
zur Bekämpfung von Botrytis.

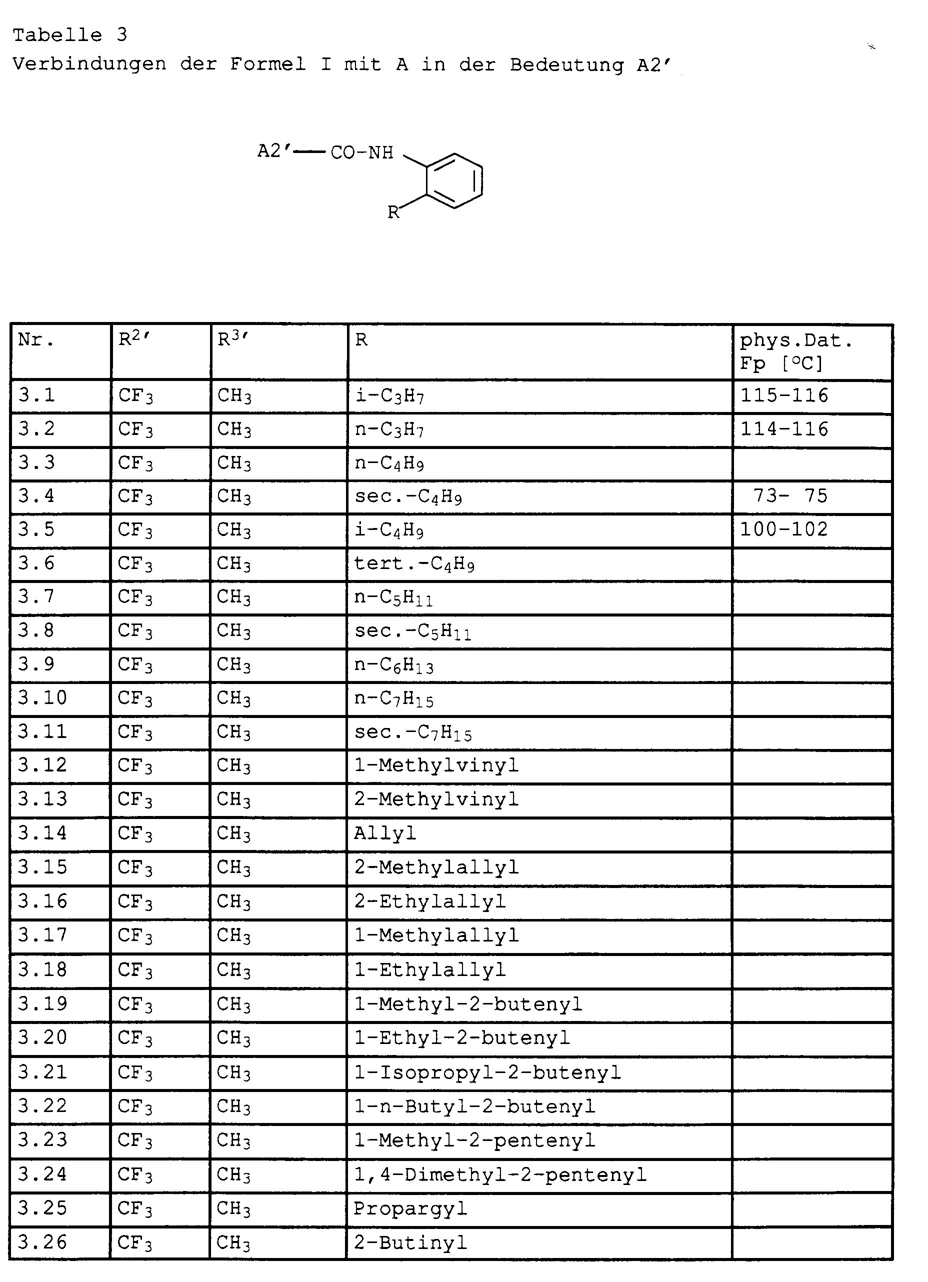

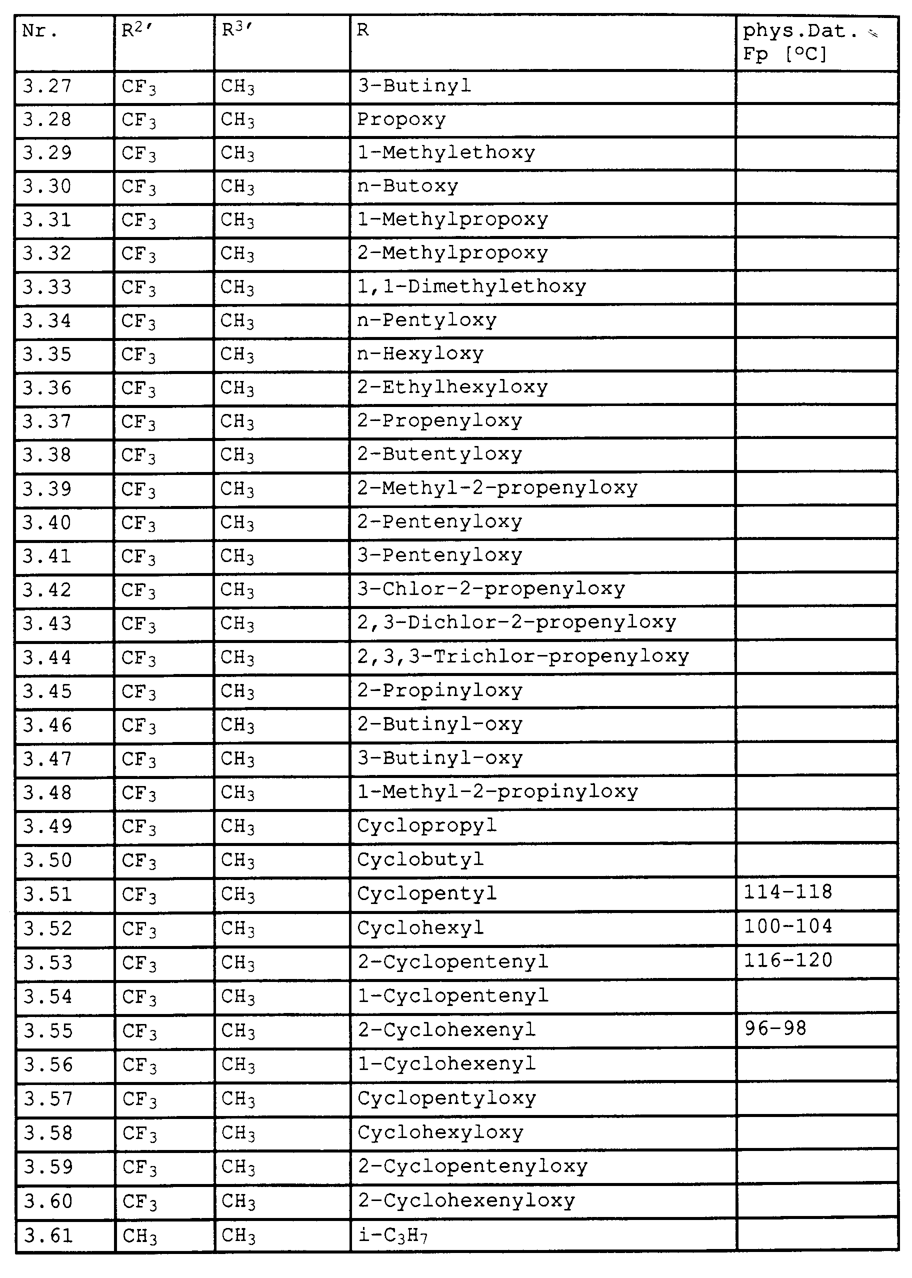

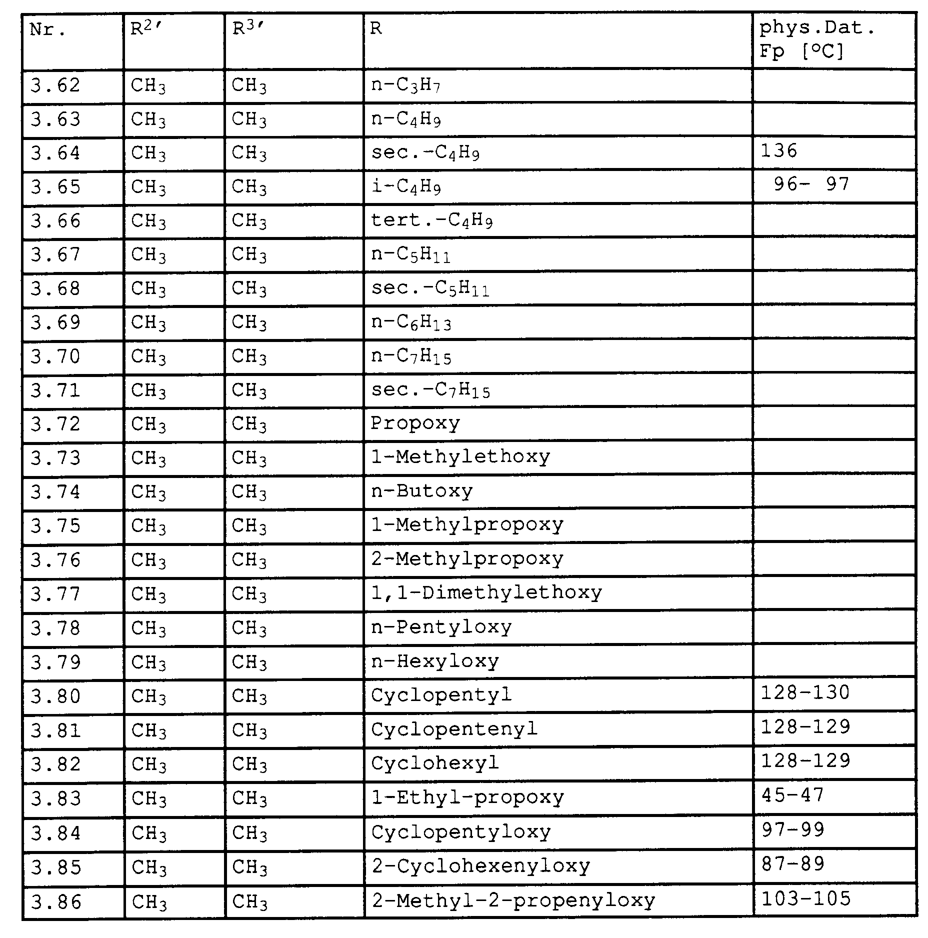

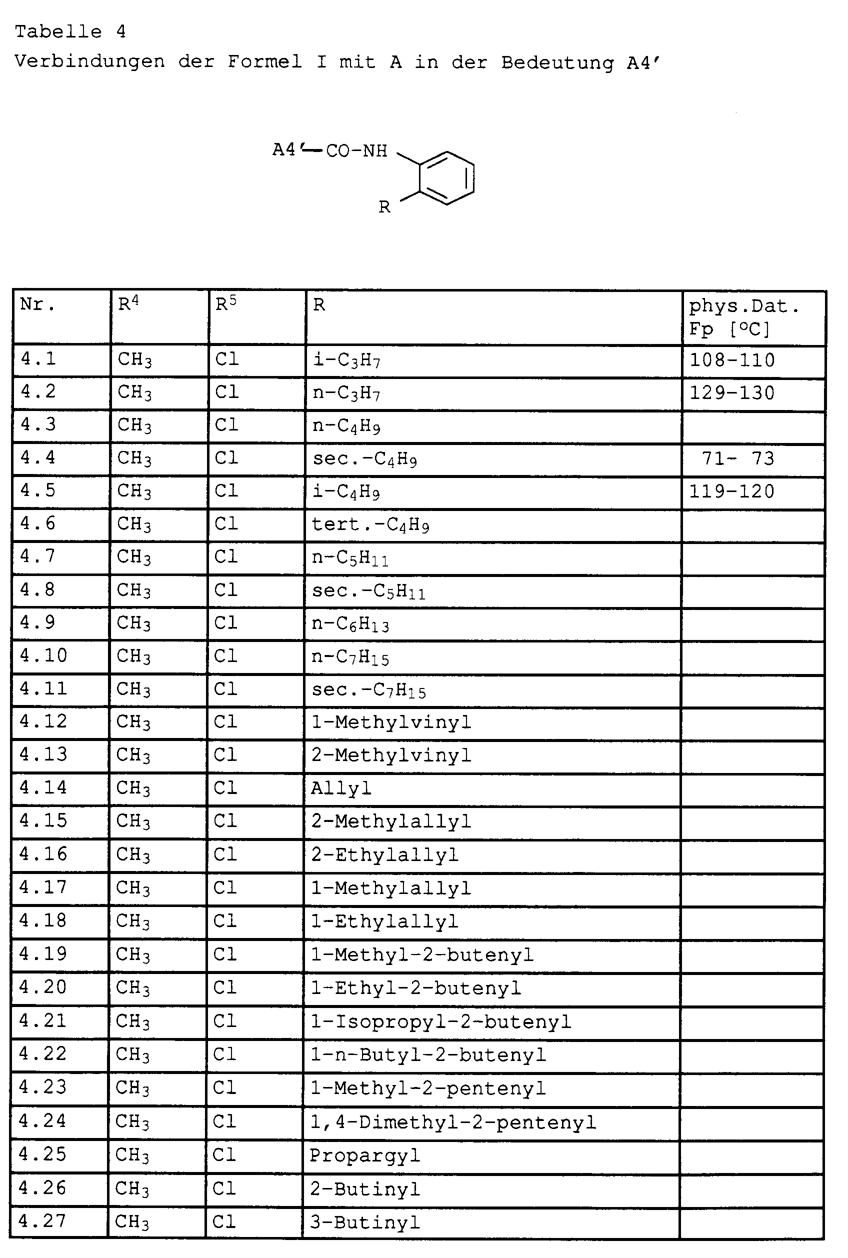

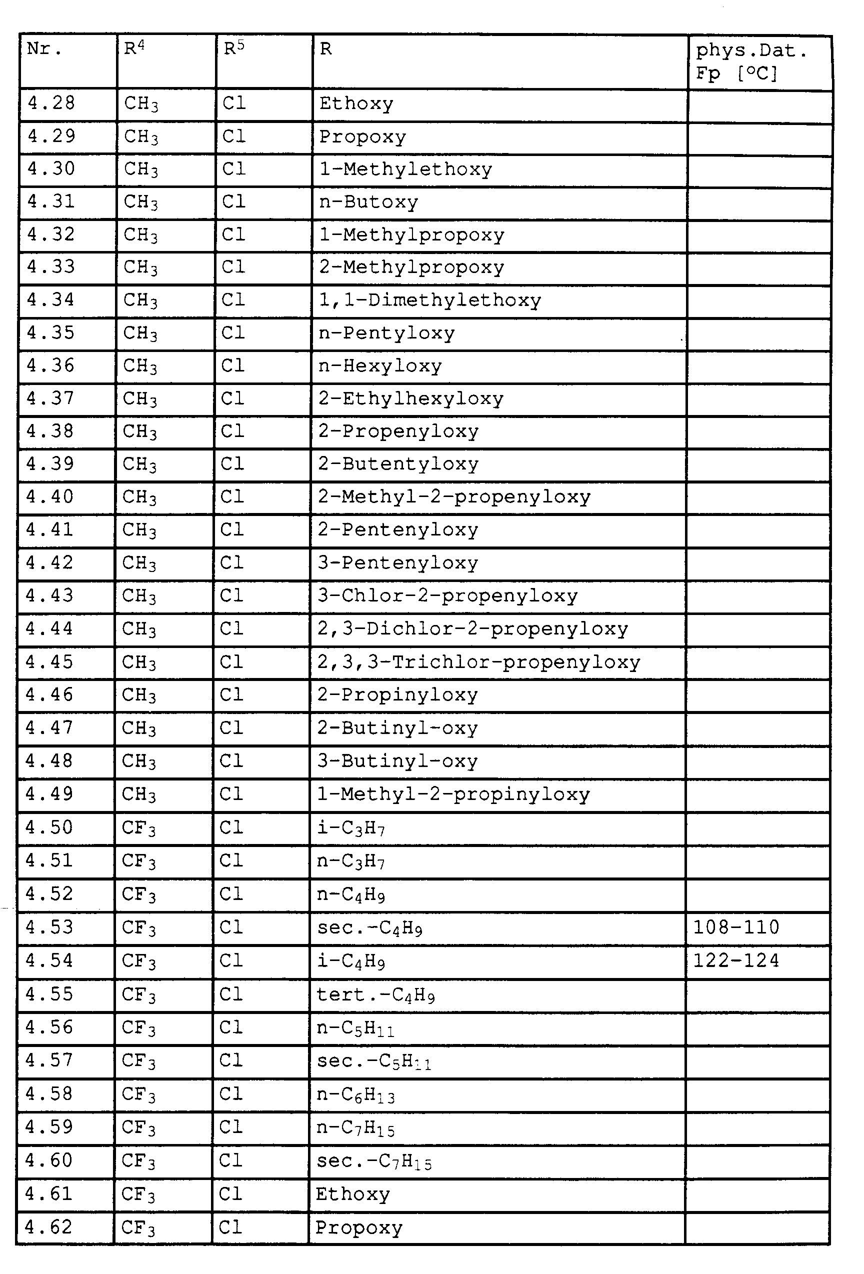

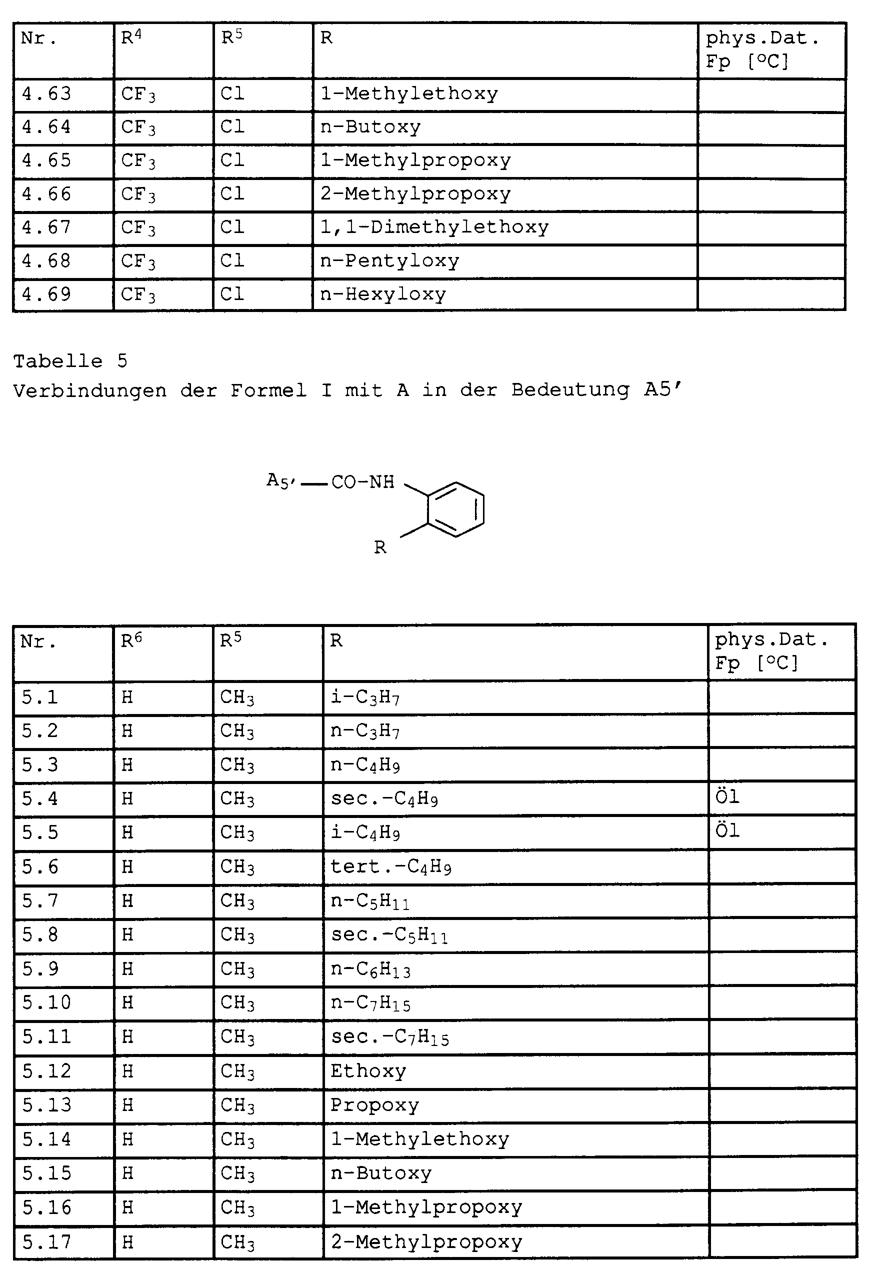

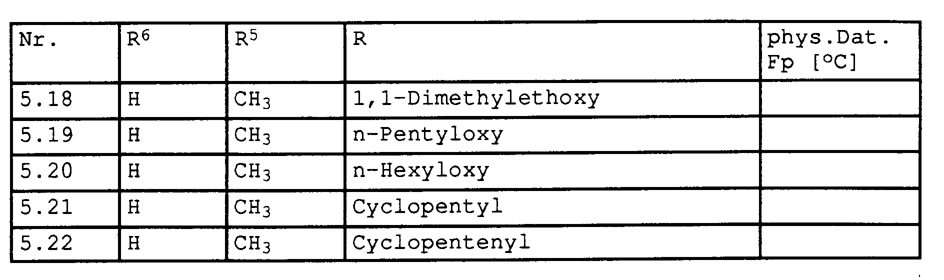

### Herstellbeispiele

### Beispiel 5

Zu einer Lösung von 1,4 g 2-n-Propylanilin und 1,1 g Triethylamin in 15 ml Tetrahydrofuran tropft man bei 0°C 2,3 g 2-Methyl-4-trifluormethyl-thiazol-5-carbonsäurechlorid und rührt noch 12 Stdn. bei 20°C.

Nach Verdünnen mit 300 ml Wasser, Extraktion mit Methyl-tert.-butylether (2x 70 ml), Verdampfen des Lösungsmittels und Mischen des Rückstandes mit wenig n-Pentan isoliert man 2,8 g 2-Methyl-4-trifluormethyl-thiazol-5-carbonsäure-2-n-propyl-anilid vom Fp.: 114-116°C (Tabelle 3, Nr. 2).

### Beispiel 6

Zu einer Lösung von 2,7 g 2-i-Propylanilin und 2,2 g Triethylamin in 40 ml Dichlormethan tropft man bei 0°C 3,8 g 1,3-Dimethyl-5-chlor-pyrazol-4-carbonsäurechlorid und rührt noch 2 Stdn. bei 0°C.

Nach Waschen mit 50 ml Wasser, Verdampfen des Lösungsmittels und Umkristallisieren aus Cyclohexan isoliert man 3,3 g 1,3-Dimethyl-5-chlor-pyrazol-4-carbonsäure-2-isopropylanilid vom Fp. 108 - 110°C (Tabelle 4, Nr. 1).

Die Erfindung betrifft ferner die folgenden neuen Verbindungen.

Carbonsäureamide der allgemeinen Formel I, in der die Reste die folgenden Bedeutungen haben:
- A: 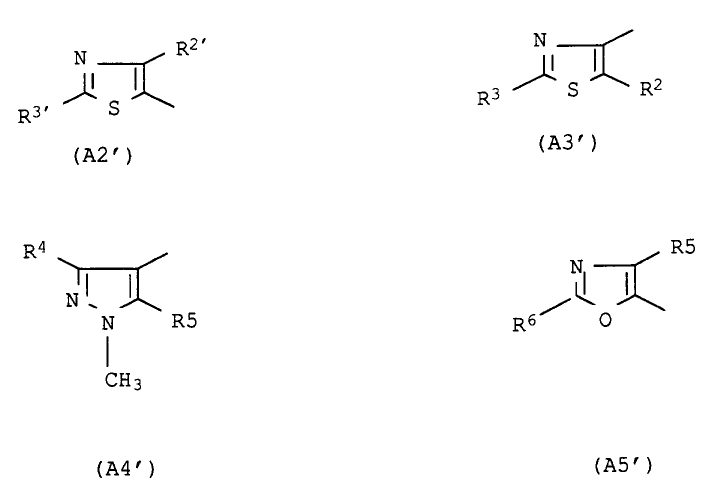
worin die Substituenten R² bis R⁶ ihrerseits bedeuten:
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;

- R: gegebenenfalls durch Halogen substituiertes C₃-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy,
ausgenommen die Verbindungen I, in denen
a)
   - A =: A2',
   - R =: gegebenenfalls durch Halogen substituiertes Ethoxy
b)
   - A =: A4',
   - R =: gegebenenfalls substituiertes: Cycloalkyl, Cycloalkenyl, Cycloalkyloxy oder Cycloalkenyloxy, und
c)
   - A =: A4'
   - R =: gegebenenfalls durch Halogen substituiertes Alkyl, Alkoxy, Alkenyl, Alkinyl.

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 1.7 und 10 Gew.-Teilen N-Methyl-a-pyrrolidon, die zur Anwendung in Form kleinster Tropfen geeignet ist;
II. eine Mischung aus 20 Gew.-Teilen der Verbindung Nr. 1.8, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1.3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1.4, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 1.5, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 1.7 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1.8, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1.9, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1.33, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen Botrytis aus. Sie sind zum Teil systemisch wirksam und können als Blatt- und Bodenfungizide eingesetzt werden.

Besondere Bedeutung haben sie für die Bekämpfung von Botrytis an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man den Pilz oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid,;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-ßbis-(dimethylamino)-phosphinyl'-3-phenyl-1,2,4-triazol,
2,3-Dicyano-1,4-dithioanthrachinon,
2-Thio-1,3-dithioloß4,5-b'chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon, Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenyl)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-83-methoxycarbonyl-2-thioureido)-benzol,
sowie verschiedene Fungizide, wie Dodecylguanidinacetat,
3-[3-(3,5-Dimethyl-2-oxycyclohexyl)-2-hydroxyethyl)]glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorhenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

### Anwendungsbeispiele

Als Vergleichswirkstoffe wurden 2-Chlornicotinsäure-2'-ethylanilid (A) - bekannt aus US-A 4 001 416 - und 2-Chlornicotinsäure-3'-isopropylanilid (B) - bekannt aus DE-A 26 11 601 - benutzt.

### Anwendungsbeispiel 1

### Wirksamkeit gegen Botrytis cinerea auf Paprikaschoten

Scheiben von grünen Paprikaschoten wurden mit wäßriger Wirkstoffaufbereitung, die 80 % Wirkstoff und 20 % Emulgiermittel in der Trockensubstanz enthielt, tropfnaß besprüht. 2 Stunden nach dem Antrocknen des Spritzbelages wurden die Fruchtscheiben mit einer Sporensuspension von Botrytis cinerea, die 1,7 x 10⁶ Sporen pro ml einer 2 %igen Biomalzlösung enthielt, behandelt. Die Fruchtscheiben wurden anschließend in feuchten Kammern bei 18°C für 4 Tage aufbewahrt. Danach erfolgte visuell die Auswertung der Botrytis-Entwicklung auf den befallenen Fruchtscheiben.

Das Ergebnis zeigt, daß die Wirkstoffe 1.5, 1.7 und 1.8 bei der Anwendung als 500 ppm haltige Spritzbrühe eine bessere fungizide Wirkung zeigen (95 %) als die bekannten Vergleichswirkstoffe A (10 %) und B (65 %).

### Anwendungsbeispiel 2

### Wirksamkeit gegen Botrytis cinerea auf Paprikaschoten

Die Innenfläche von aufgeschnittenen Paprikaschoten wurde mit einer wäßrigen Wirkstoffaufbereitung, die 80 % Wirkstoff und 20 % Emulgiermittel in der Trockensubstanz enthielt, bis zur Tropfnässe besprüht. Nach dem Antrocknen der wäßrigen Wirkstoffaufbereitung wurden die Fruchtstücke mit einer wäßrigen Sporensuspension von Botrytis cinerea, die 1,7 x 10⁶ Sporen/ml enthielt, inokuliert.

Anschließend wurden die Fruchtstücke für 4 Tage in Klimaschränke bei 20 - 22°C gestellt. Dann wurde das Ausmaß des Pilzbewuchses visuell ausgewertet.

Das Ergebnis des Versuchs zeigt ferner, daß die Verbindungen Nr. 3.1, 3.2, 3.4, 3.5, 4.1, 4.2, 4.4, 4.5 bei der Anwendung als 1000 ppm Wirkstoff enthaltende wäßrige Spritzbrühen eine gute fungizide Wirkung (100 %) haben.

## Patentansprüche

1. Verwendung von Carbonsäureamiden der allgemeinen Formel I 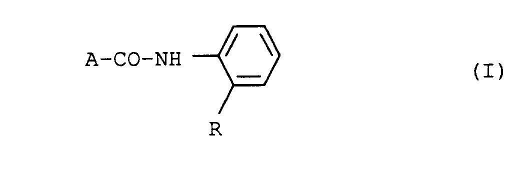 in der die Reste die folgenden Bedeutungen haben:
A
A1: Pyridin-3-yl, substituiert in 2-Stellung durch Halogen, Methyl, Trifluormethyl, Methoxy, Methylthio, Methylsulfinyl, Methylsulfonyl,
A2: Thiazol-5-yl, substituiert in 2- und 4-Stellung durch Wasserstoff, Methyl, Trifluormethyl,
A3: Thiazol-4-yl, substituiert in 2- und 5-Stellung durch Wasserstoff, Methyl, Chlor, Trifluormethyl,
A4: 1-Methylpyrazol-4-yl, substituiert in 3-Stellung durch Methyl, Chlor, Trifluormethyl und in 5-Stellung durch Chlor, oder
A5: Oxazol-5-yl, substituiert in 2- und 4-Stellung durch Wasserstoff, Methyl, Chlor, und
R gegebenenfalls durch Halogen substituiertes C₂-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy,
gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy, gegebenenfalls durch C₁-C₄-Alkyl, C₁-C₄-Alkoxy, C₁-C₄-Alkylthio, Halogen, substituiertes Phenyl,
ausgenommen die Verbindungen I, in denen
a)
A = A1, welches in 2-Stellung Halogen trägt,
R = gegebenenfalls durch Halogen substituiertes Ethyl oder gegebenenfalls durch Halogen substituiertes Ethoxy
b)
A = A1, welches in 2-Stellung Halogen trägt,
R = unsubstituiertes Phenyl
c)
A = A2,
R = gegebenfalls durch Halogen substituiertes Ethyl und gegebenenfalls durch Halogen substituiertes Ethoxy
d)
A = A2,
R = unsubstituiertes Phenyl
e)
A = A4,
R = gegebenenfalls substituiertes: Cycloalkyl, Cycloalkenyl, Cycloalkyloxy oder Cycloalkenyloxy und
f)
A = A4,
R = gegebenenfalls durch Halogen substituiertes Ethyl,
zur Bekämpfung von Botrytis.

2. Verwendung nach Anspruch 1 von Carbonsäureamiden der allgemeinen Formel I gemäß Anspruch 1, in der die Reste die folgenden Bedeutungen haben:
A 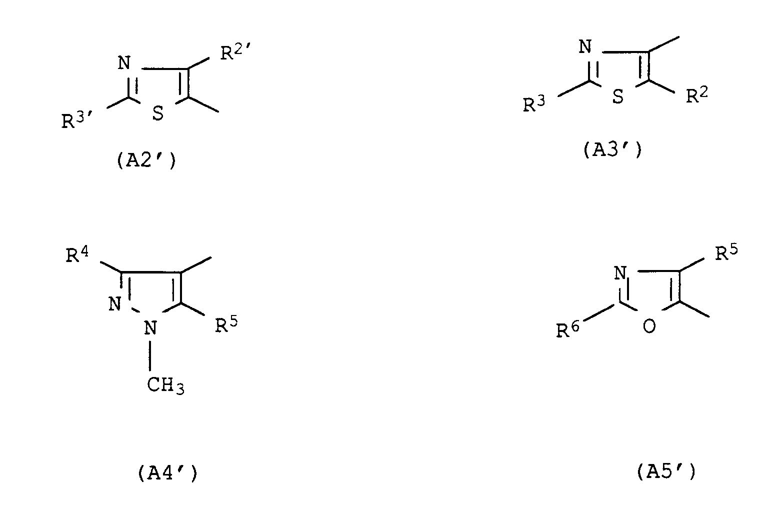
worin die Substituenten R² bis R⁶ ihrerseits bedeuten:
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;
R gegebenenfalls durch Halogen substituiertes C₃-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy,
ausgenommen die Verbindungen I, in denen
a)
A = A2',
R = gegebenenfalls durch Halogen substituiertes Ethoxy und
b)
A = A4',
R = gegebenenfalls substituiertes: Cycloalkyl, Cycloalkenyl, Cycloalkyloxy oder Cycloalkenyloxy.

3. Verwendung nach Anspruch 1 von Carbonsäureamiden der allgemeinen Formel I gemäß Anspruch 1, in der die Reste die folgenden Bedeutungen haben:
A 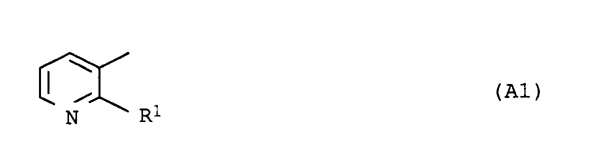
R¹ Halogen, Methyl, Trifluormethyl, Methoxy, Methylthio, Methylsulfinyl, Methylsulfonyl und
R gegebenenfalls durch Halogen substituiertes C₂-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, C₃-C₆-Cycloalkyl, C₄-C₆-Cycloalkenyl, C₅-C₆-Cycloalkyloxy, C₅-C₆-Cycloalkenyloxy,
ausgenommen die Verbindungen I, in denen
A = A1, welches in 2-Stellung Halogen trägt,
R = gegebenenfalls durch Halogen substituiertes Ethyl oder gegebenenfalls durch Halogen substituiertes Ethoxy.

4. Verwendung nach Anspruch 1 von Carbonsäureamiden der allgemeinen Formel I gemäß Anspruch 1, in der die Reste die folgenden Bedeutungen haben:
A 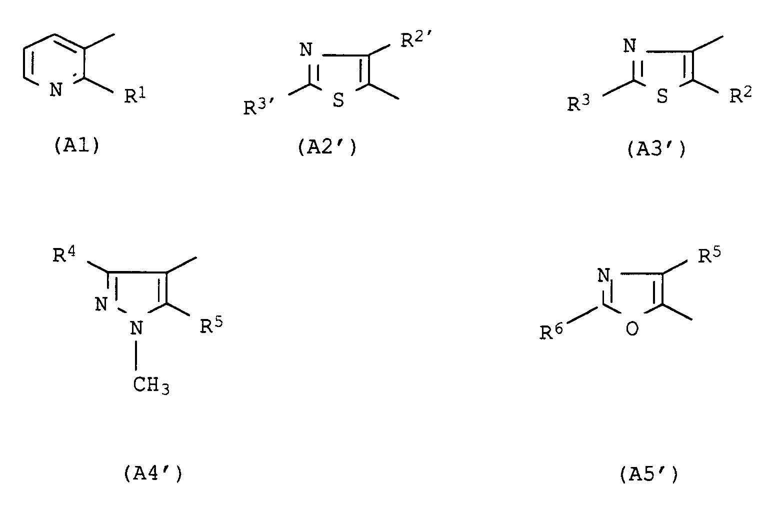
worin die Substituenten R¹ bis R⁶ ihrerseits bedeuten:
R¹ Trifluormethyl, Chlor,
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;
R Phenyl, welches einfach substituiert sein kann durch C₁-C₄-Alkyl, C₁-C₄-Alkoxy, C₁-C₄-Alkylthio, Halogen,
ausgenommen die Verbindungen I, in denen
a)
A = A1, welches in 2-Stellung Halogen trägt,
R = unsubstituiertes Phenyl und
b)
A = A2',
R = unsubstituiertes Phenyl.

5. Carbonsäureamide der allgemeinen Formel I gemäß Anspruch 1, in der die Reste die folgenden Bedeutungen haben:
A 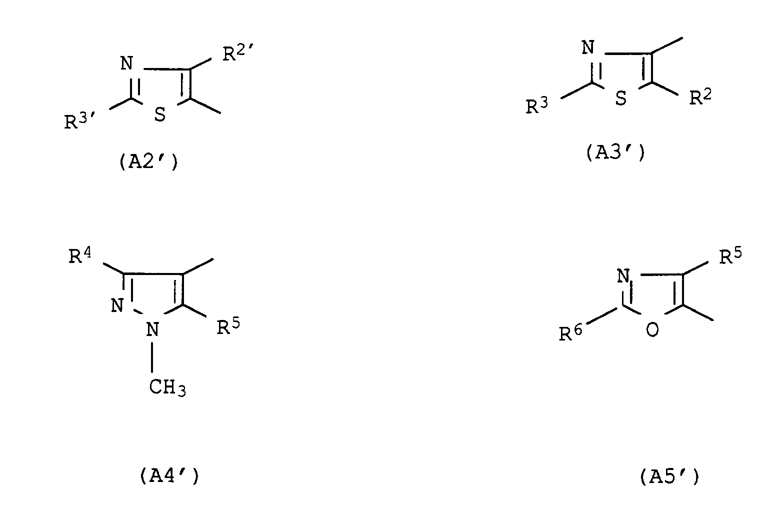
worin die Substituenten R² bis R⁶ ihrerseits bedeuten:
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;
R gegebenenfalls durch Halogen substituiertes C₃-C₁₂-Alkyl, gegebenenfalls durch Halogen substituiertes C₃-C₁₂-Alkenyl, C₃-C₆-Alkinyl, gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, gegebenenfalls durch C₁-C₄-Alkyl substituiertes C₃-C₆-Cycloalkyl, gegebenenfalls durch C₁-C₄-Alkyl substituiertes C₄-C₆-Cycloalkenyl, gegebenenfalls durch C₁-C₄-Alkyl substituiertes C₅-C₆-Cycloalkyloxy, gegebenenfalls durch C₁-C₄-Alkyl substituiertes C₅-C₆-Cycloalkenyloxy,
ausgenommen die Verbindungen I, in denen
a)
A = A2',
R = gegebenenfalls durch Halogen substituiertes Ethoxy und
b)
A = A4',
R = gegebenenfalls substituiertes: Cycloalkyl, Cycloalkenyl, Cycloalkyloxy oder Cycloalkenyloxy, und
c)
A = A4'
R = gegebenenfalls durch Halogen substituiertes Alkyl, Alkoxy, Alkenyl, Alkinyl.

6. Carbonsäureamide der allgemeinen Formel I gemäß Anspruch 1, in der die Reste die folgenden Bedeutungen haben: 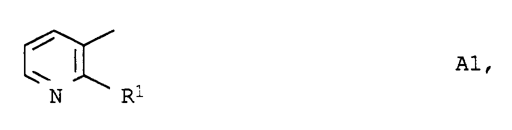 worin
R¹ Halogen, Methyl, Trifluormethyl, Methoxy, Methylsulfinyl, Methylsulfonyl,
R gegebenenfalls durch Halogen substituiertes C₃-C₁₂-Alkyl, gegebenenfalls durch Halogen substituiertes C₃-C₁₂-Alkenyl, C₃-C₆-Alkinyl gegebenenfalls durch Halogen substituiertes C₂-C₁₂-Alkoxy, gegebenenfalls durch Halogen substituiertes C₃-C₁₂-Alkenyloxy, C₃-C₁₂-Alkinyloxy, C₃-C₆-Cycloalkyl, C₄-C₆-Cycloalkenyl, C₅-C₆-Cycloalkyloxy, C₅-C₆-Cycloalkenyloxy,
ausgenommen die Verbindungen I, in denen
A = A1, welches in 2-Stellung Chlor trägt,
R = iso-Propyl und
A = A1, welches in 2-Stellung Halogen trägt,
R = gegebenenfalls durch Halogen substituiertes Ethoxy.

7. Carbonsäureamide der allgemeinen Formel I gemäß Anspruch 1, in der die Reste die folgenden Bedeutungen haben:
A 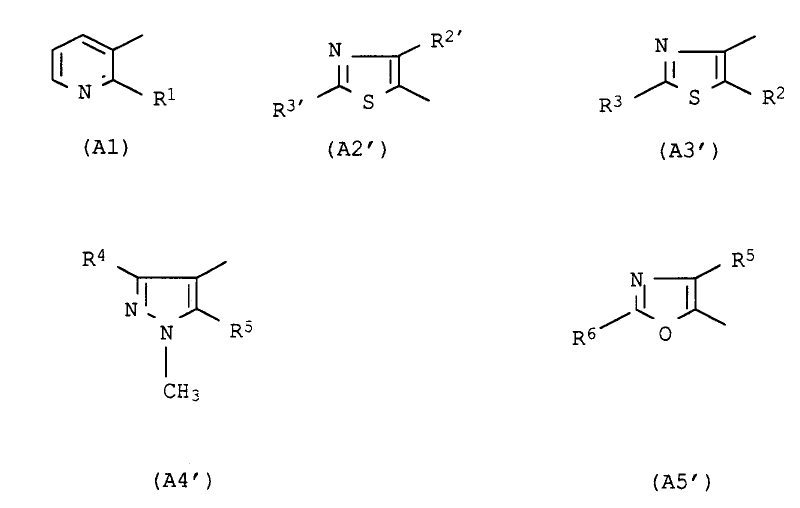
worin die Substituenten R¹ bis R⁶ ihrerseits bedeuten:
R¹ Trifluormethyl, Chlor,
R² Methyl, Trifluormethyl, Chlor,
R^{2'} Methyl, Trifluormethyl,
R³ Wasserstoff, Methyl, Chlor,
R^{3'} Wasserstoff, Methyl,
R⁴ Methyl, Trifluormethyl,
R⁵ Chlor,
R⁶ Wasserstoff oder Methyl;
R Phenyl, welches einfach substituiert sein kann durch C₁-C₄-Alkyl, C₁-C₄-Alkoxy, C₁-C₄-Alkylthio, Halogen
ausgenommen die Verbindungen I, in denen
a)
A = A1, welches in 2-Stellung Halogen trägt,
R = unsubstituiertes Phenyl
b)
A = A2',
R = unsubstituiertes Phenyl und
c)
A = A4',
R = unsubstituiertes Phenyl.

8. Botrytizides Mittel, enthaltend eine botrytizid wirksame Menge mindestens einer der Verbindungen I gemäß den Ansprüchen 5 bis 7 und einen festen oder flüssigen Trägerstoff.

## Claims

1. The use of carboxamides of the general formula I 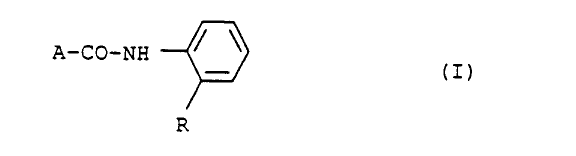 where the radicals have the following meanings:
A is
A1: pyridin-3-yl, substituted in the 2-position by halogen, methyl, trifluoromethyl, methoxy, methylthio, methylsulfinyl, methylsulfonyl,
A2: thiazol-5-yl, substituted in the 2- and 4-position by hydrogen, methyl, trifluoromethyl,
A3: thioazol-4-yl, substituted in the 2- and 5-position by hydrogen, methyl, chlorine, trifluoromethyl,
A4: 1-methylpyrazol-4-yl, substituted in the 3-position by methyl, chlorine, trifluoromethyl and in the 5-position by chlorine, or
A5: oxazol-5-yl, substituted in the 2- and 4-position by hydrogen, methyl, chlorine, and
R is C₂-C₁₂-alkyl, unsubstituted or substituted by halogen, C₃-C₁₂-alkenyl, unsubstituted or substituted by halogen, C₃-C₆-alkynyl, C₂-C₁₂-alkoxy, unsubstituted or substituted by halogen, C₃-C₁₂-alkenyloxy, unsubstituted or substituted by halogen, or C₃-C₁₂-alkynyloxy,
C₃-C₆-cycloalkyl, unsubstituted or substituted by C₁-C₄-alkyl, C₄-C₆-cycloalkenyl, unsubstituted or substituted by C₁-C₄-alkyl, C₅-C₆-cycloalkyloxy, unsubstituted or substituted by C₁-C₄-alkyl, C₅-C₆-cycloalkenyloxy, unsubstituted or substituted by C₁-C₄-alkyl, phenyl, unsubstituted or substituted by C₁-C₄-alkyl, C₁-C₄-alkoxy, C₁-C₄-alkylthio or halogen,
with the exception of the compounds I where
a)
A = A1 which has halogen attached to it in the 2-position,
R = ethyl which is unsubstituted or substituted by halogen or ethoxy which is unsubstituted or substituted by halogen,
b)
A = A1 which has halogen attached to it in the 2-position
R = unsubstituted phenyl
c)
A = A2,
R = ethyl which is unsubstituted or substituted by halogen and ethoxy which is unsubstituted or substituted by halogen,
d)
A = A2,
R = unsubstituted phenyl
e)
A = A4,
R = unsubstituted or substituted: cycloalkyl, cycloalkenyl, cycloalkoxy or cycloalkenyloxy and
f)
A = A4,
R = ethyl, unsubstituted or substituted by halogen,
for controlling botrytis.

2. The use as claimed in claim 1 of carboxamides of the general formula I as claimed in claim 1 where the radicals have the following meanings:
A is 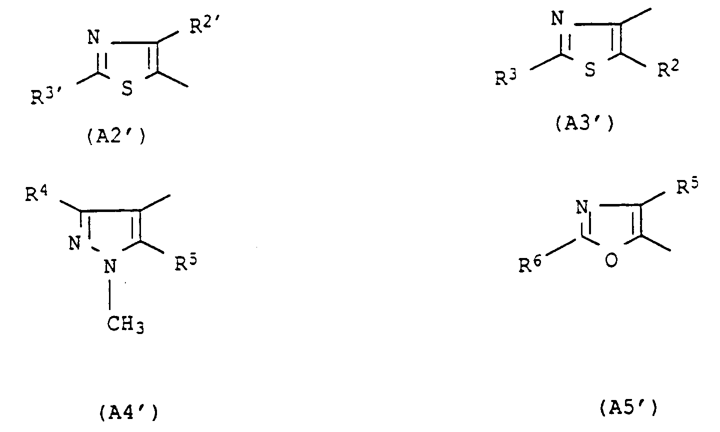
where the substituents R² to R⁶, in turn, have the following meanings:
R² is methyl, trifluoromethyl, chlorine,
R^{2'} is methyl, trifluoromethyl,
R³ is hydrogen, methyl, chlorine,
R^{3'} is hydrogen, methyl,
R⁴ is methyl, trifluoromethyl,
R⁵ is chlorine,
R⁶ is hydrogen or methyl;
R is C₃-C₁₂-alkyl, unsubstituted or substituted by halogen, C₃-C₁₂-alkenyl, unsubstituted or substituted by halogen, C₃-C₆-alkynyl, C₂-C₁₂-alkoxy, unsubstituted or substituted by halogen, C₃-C₁₂-alkenyloxy, unsubstituted or substituted by halogen, C₃-C₁₂-alkynyloxy, C₃-C₆-cycloalkyl, unsubstituted or substituted by C₁-C₄-alkyl, C₄-C₆-cycloalkenyl, unsubstituted or substituted by C₁-C₄-alkyl, C₅-C₆-cycloalkyloxy, unsubstituted or substituted by C₁-C₄-alkyl, C₅-C₆-cycloalkenyloxy, unsubstituted or substituted by C₁-C₄-alkyl,
with the exception of the compounds I where
a)
A = A2',
R = ethoxy, unsubstituted or substituted by halogen, and
b)
A = A4',
R = in each case unsubstituted or substituted: cycloalkyl, cycloalkenyl, cycloalkyloxy or cycloalkenyloxy.

3. The use as claimed in claim 1 of carboxamides of the general formula I as claimed in claim 1, where the radicals have the following meanings:
A is 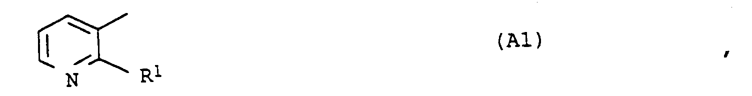
R¹ is halogen, methyl trifluoromethyl, methoxy, methylthio, methylsulfinyl, methylsulfonyl and
R is C₂-C₁₂-alkyl, unsubstituted or substituted by halogen, C₃C₁₂-alkenyl, unsubstituted or substituted by halogen, C₃-C₆-alkynyl, C₂-C₁₂-alkoxy, unsubstituted or substituted by halogen, C₃-C₁₂-alkenyloxy, unsubstituted or substituted by halogen, C₃-C₁₂-alkynyloxy, C₃-C₆-cycloalkyl, C₄-C₆-cycloalkenyl, C₅-C₆-cycloalkyloxy, C₅-C₆-cycloalkenyloxy,
with the exception of the compounds I, where
A = A1 which has halogen attached to it in the 2-position,
R = ethyl, unsubstituted or substituted by halogen, or ethoxy, unsubstituted or substituted by halogen.

4. The use as claimed in claim 1 of carboxamides of the general formula I as claimed in claim 1 where the radicals have the following meanings:
A is 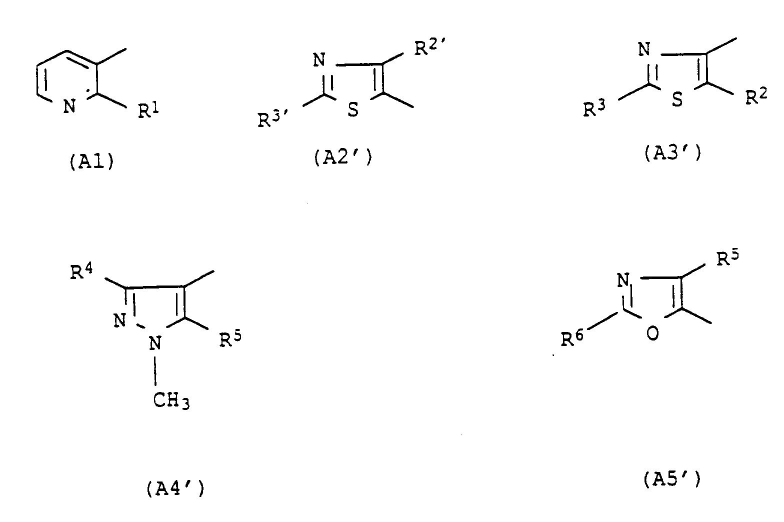
where the substituents R¹ to R⁶, in turn, have the following meanings:
R¹ is trifluoromethyl, chlorine,
R² is methyl, trifluoromethyl, chlorine,
R^{2'} is methyl, trifluoromethyl,
R³ is hydrogen, methyl, chlorine,
R^{3'} is hydrogen, methyl,
R⁴ is methyl, trifluoromethyl,
R⁵ is chlorine,
R⁶ is hydrogen or methyl;
R is phenyl which can be monosubstituted by C₁-C₄-alkyl, C₁-C₄-alkoxy, C₁-C₄-alkylthio, halogen,
with the exception of the compounds I where
a)
A = A1, which has halogen attached to it in the 2-position,
R = unsubstituted phenyl and
b)
A = A2',
R = unsubstituted phenyl.

5. A carboxamide of the general formula I as claimed in claim 1, where the radicals have the following meanings:
A is 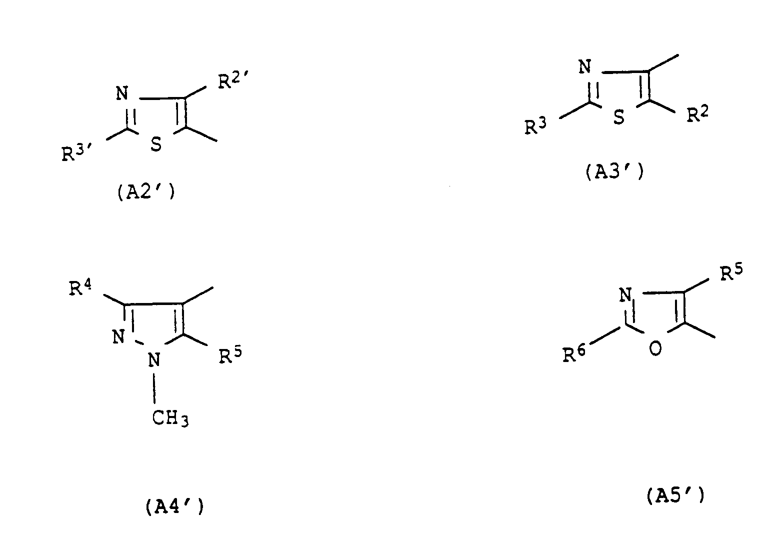
where the substituents R² to R⁶, in turn, have the following meanings:
R² is methyl, trifluoromethyl, chlorine,
R^{2'} is methyl, trifluoromethyl,
R³ is hydrogen, methyl, chlorine,
R^{3'} is hydrogen, methyl,
R⁴ is methyl, trifluoromethyl,
R⁵ is chlorine,
R⁶ is hydrogen or methyl;
R is C₃-C₁₂-alkyl, unsubstituted or substituted by halogen, C₃-C₁₂-alkenyl, unsubstituted or substituted by halogen, C₃-C₆-alkynyl, C₂-C₁₂-alkoxy, unsubstituted or substituted by halogen, C₃-C₁₂-alkenyloxy, unsubstituted or substituted by halogen, C₃-C₁₂-alkynyloxy, C₃-C₆-cycloalkyl, unsubstituted or substituted by C₁-C₄-alkyl, C₄-C₆-cycloalkenyl, unsubstituted or substituted by C₁-C₄-alkyl, C₅-C₆-cycloalkyloxy, unsubstituted or substituted by C₁-C₄-alkyl, C₅-C₆-cycloalkenyloxy, unsubstituted or substituted by C₁-C₄-alkyl,
with the exception of the compounds I where
a)
A = A2',
R = ethoxy, unsubstituted or substituted by halogen, and
b)
A = A4',
R = in each case unsubstituted or substituted: cycloalkyl, cycloalkenyl, cycloalkyloxy or cycloalkenyloxy, and
c)
A = A4'
R = alkyl, alkoxy, alkenyl, alkynyl, in each case unsubstituted or substituted by halogen.

6. A carboxamide of the general formula I as claimed in claim 1 where the radicals have the following meanings: 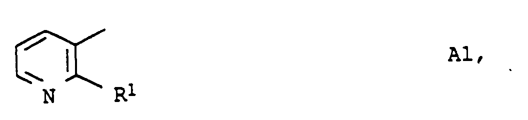 where
R¹ is halogen, methyl, trifluoromethyl, methoxy, methylsulfinyl, methylsulfonyl,
R is C₃-C₁₂-alkyl, unsubstituted or substituted by halogen, C₃-C₁₂-alkenyl, unsubstituted or substituted by halogen, C₃-C₆-alkynyl, C₂-C₁₂-alkoxy, unsubstituted or substituted by halogen, C₃-C₁₂-alkenyloxy, unsubstituted or substituted by halogen, C₃-C₁₂-alkynyloxy, C₃-C₆-cycloalkyl, C₄-C₆-cycloalkenyl, C₅-C₆-cycloalkyloxy, C₅-C₆-cycloalkenyloxy,
with the exception of the compounds I where
A = A1, which has chlorine attached to it in the 2-position,
R = isopropyl and
A = A1 which has halogen attached to it in the 2-position,
R = ethoxy, unsubstituted or substituted by halogen,

7. A carboxamide of the general formula I as claimed in claim 1 where the radicals have the following meanings: 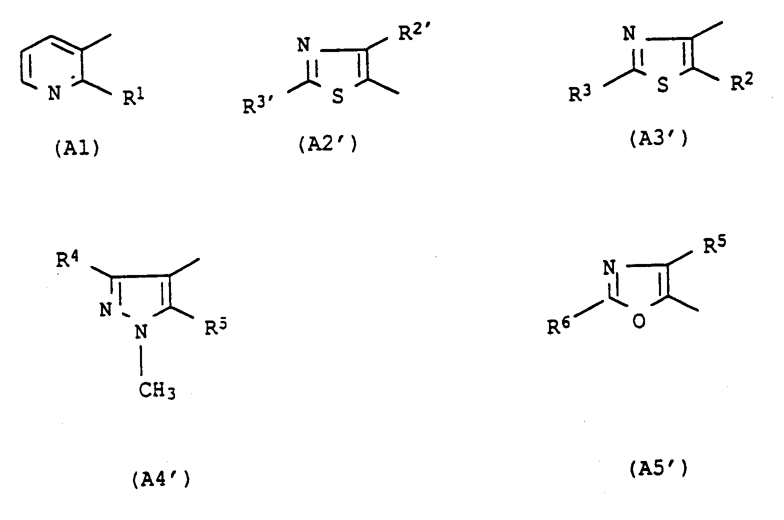 where the substituents R¹ to R⁶, in turn, have the following meanings:
R¹ is trifluoromethyl, chlorine,
R² is methyl, trifluoromethyl, chlorine,
R^{2'} is methyl, trifluoromethyl,
R³ is hydrogen, methyl, chlorine,
R^{3'} is hydrogen, methyl,
R⁴ is methyl, trifluoromethyl,
R⁵ is chlorine,
R⁶ is hydrogen or methyl;
R is phenyl which can be monosubstituted by C₁-C₄-alkyl, C₁-C₄-alkoxy, C₁-C₄-alkylthio, halogen,
with the exception of the compounds I where
a)
A = A1, which has halogen attached to it in the 2-position,
R = unsubstituted phenyl,
b)
A = A2',
R = unsubstituted phenyl and
c)
A = A4'
R = unsubstituted phenyl.

8. A botryticidal composition comprising a botryticidally active amount of at least one of the compounds I as claimed in any of claims 5 to 7 and a solid or liquid carrier.

## Revendications

1. Utilisation d'amides d'acides carboxyliques de formule générale I 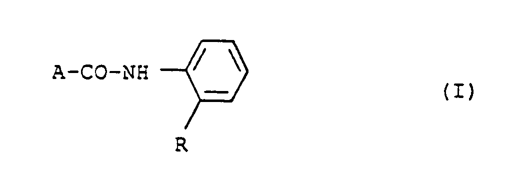 où les restes représentent les groupes suivants:
A
A1: pyridin-3-yle, substitué en position 2 par un radical halogéno, méthyle, trifluorométhyle, méthoxy, méthylthio, méthylsulfinyle, méthylsulfonyle,
A2: thiazol-5-yle, substitué en position 2 et en position 4 par un radical hydrogène, méthyle, trifluorométhyle,
A3: thiazol-4-yle, substitué en position 2 et en position 5 par un radical hydrogène, méthyle, chlore, trifluorométhyle,
A4: 1-méthylpyrazol-4-yle, substitué en position 3 par un radical méthyle, chloro, trifluorométhyle et en position 5 par un radical chloro, ou
A5: oxazol-5-yle, substitué en position 2 et en position 4 par un radical hydrogène, méthyle, chloro, et
R alkyle en C₂-C₁₂ éventuellement substitué par un halogène, alcényle en C₃-C₁₂ éventuellement substitué par un halogène, alcynyle en C₃-C₆, alcoxy en C₂-C₁₂ éventuellement substitué par un halogène, alcényloxy en C₃-C₁₂ éventuellement substitué par un halogène, alcynyloxy en C₃-C₁₂,
cycloalkyle en C₃-C₆ éventuellement substitué par un groupe alkyle en C₁-C₄, cycloalcényle en C₄-C₆ éventuellement substitué par un groupe alkyle en C₁-C₄, cycloalcoxy en C₅-C₆ éventuellement substitué par un radical alkyle en C₁-C₄, cycloalcényloxy en C₅-C₆ éventuellement substitué par un groupe alkyle en C₁-C₄, phényle éventuellement substitué par un groupe alkyle en C₁-C₄, alcoxy en C₁-C₄, alkylthio en C₁-C₄, halogéno,
à l'exception des composés I dans lesquels
a)
A = A1, qui porte un halogène en position 2,
R = éthyle éventuellement substitué par un halogène ou éthoxy éventuellement substitué par un halogène
b)
A = A1, qui porte un halogène en position 2,
R = phényle non-substitué,
c)
A = A2,
R = éthyle éventuellement substitué par un halogène et éthoxy éventuellement substitué par un halogène
d)
A = A2,
R = phényle non-substitué
e)
A = A4,
R = cycloalkyle, cycloalcényle, cycloalkyloxy ou cycloalcényloxy, éventuellement substitués, et
f)
A = A4,
R = éthyle éventuellement substitué par un halogène,
pour la lutte contre le botrytis.

2. Utilisation selon la revendication 1 d'amides d'acides carboxyliques de formule générale I selon la revendication 1, dans laquelle les restes représentent les groupes suivants:
A 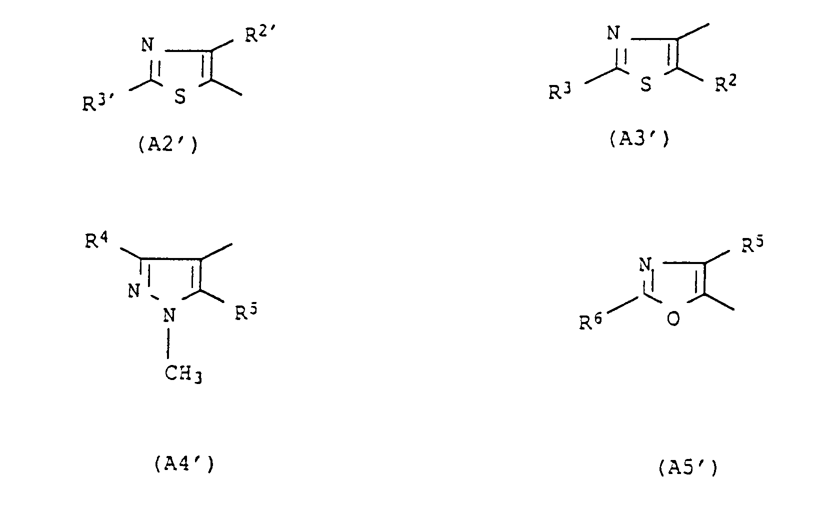 où les substituants R² à R⁶ représentent quant à eux les groupes:
R² méthyle, trifluorométhyle, chloro,
R^{2'} méthyle, trifluorométhyle,
R³ hydrogène, méthyle, chloro,
R^{3'} hydrogène, méthyle,
R⁴ méthyle, trifluorométhyle,
R⁵ chloro,
R⁶ hydrogène ou méthyle;
R alkyle en C₃-C₁₂ éventuellement substitué par un radical halogène, alcényle en C₃-C₁₂ éventuellement substitué par un halogène, alcynyle en C₃-C₆, alcoxy en C₂-C₁₂ éventuellement substitué par un halogène, alcényloxy en C₃-C₁₂ éventuellement substitué par un halogène, alcynyloxy en C₃-C₁₂, cycloalkyle en C₃-C₆ éventuellement substitué par un radical alkyle en C₁-C₄, cycloalcényle en C₄-C₆ éventuellement substitué par un radical alkyle en C₁-C₄, cycloalkyloxy en C₅-C₆ éventuellement substitué par un radical alkyle en C₁-C₄, cycloalcényloxy en C₅-C₆ éventuellement substitué par un radical alkyle en C₁-C₄,
à l'exception des composés I dans lesquels
a)
A = A2',
R = éthoxy éventuellement substitué par un halogène et
b)
A = A4',
R = cycloalkyle, cycloalcényle, cycloalkyloxy ou cycloalcényloxy, éventuellement substitués.

3. Utilisation selon la revendication 1 d'amides d'acides carboxyliques de formule générale I selon la revendication 1, dans laquelle les restes représentent les groupes suivants:
A 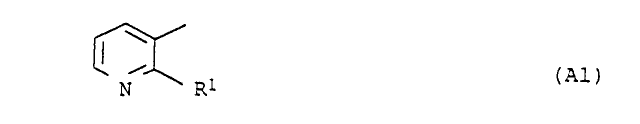
R¹ halogéno, méthyle, trifluorométhyle, méthoxy, méthylthio, méthylsulfinyle, méthylsulfonyle et
R alkyle en C₂-C₁₂ éventuellement substitué par un halogène, alcényle en C₃-C₁₂ éventuellement substitué par un halogène, alcynyle en C₃-C₆, alcoxy en C₂-C₁₂ éventuellement substitué par un halogène, alcényloxy en C₃-C₁₂ éventuellement substitué par un halogène, alcynyloxy en C₃-C₁₂, cycloalkyle en C₃-C₆, cycloalcényle en C₄-C₆, cycloalkyloxy en C₅-C₆, cycloalcényloxy en C₅-C₆,
à l'exception des composés I dans lesquels
A = A1, qui porte un halogène en position 2,
R = éthyle éventuellement substitué par un halogène ou éthoxy éventuellement substitué par un halogène.

4. Utilisation selon la revendication 1 d'amides d'acides carboxyliques de formule générale I selon la revendication 1, dans laquelle les restes représentent les groupes suivants:
A 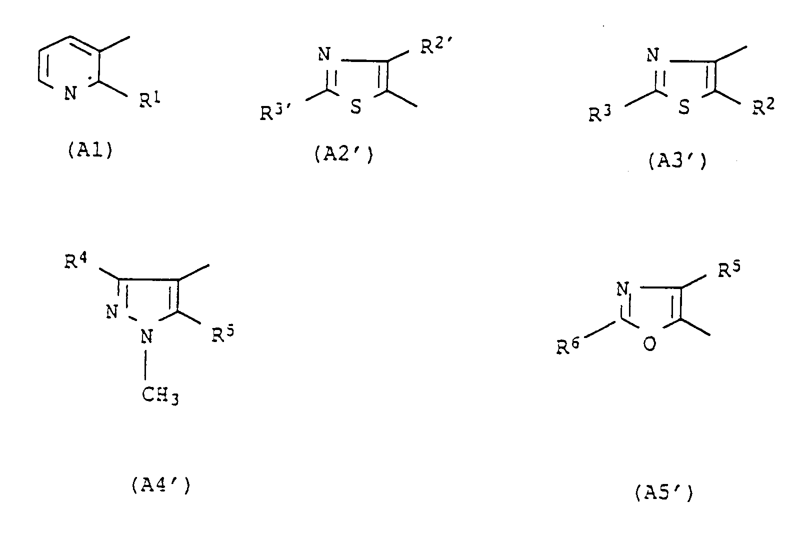 où les substituants R¹ à R⁶ représentent quant à eux les groupes:
R¹ trifluorométhyle, chloro,
R² méthyle, trifluorométhyle, chloro,
R^{2'} méthyle, trifluorométhyle,
R³ hydrogène, méthyle, chloro,
R^{3'} hydrogène, méthyle,
R⁴ méthyle, trifluorométhyle,
R⁵ chloro,
R⁶ hydrogène ou méthyle;
R phényle, qui peut être substitué une fois par un groupe alkyle en C₁-C₄, alcoxy en C₁-C₄, alkylthio en C₁-C₄, halogéno,
à l'exception des composés I dans lesquels
a)
A = A1, qui porte un halogène en position 2,
R = phényle non-substitué et
b)
A = A2',
R = phényle non-substitué.

5. Amides d'acides carboxyliques de formule générale I selon la revendication 1, dans lesquels les restes représentent les groupes suivants:
A 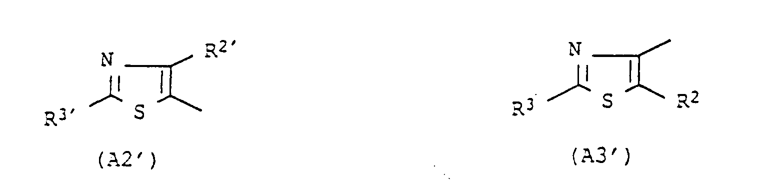 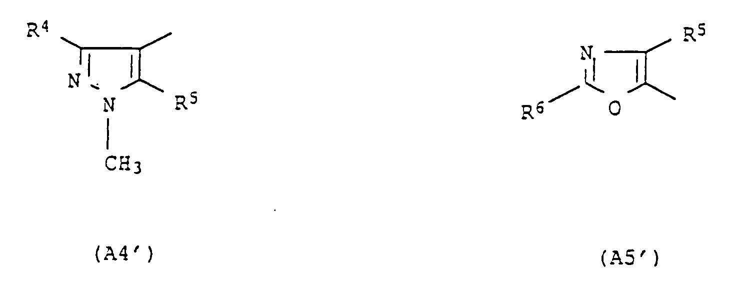 où les substituants R² à R⁶ représentent quant à eux les groupes:
R² méthyle, trifluorométhyle, chloro,
R^{2'} méthyle, trifluorométhyle,
R³ hydrogène, méthyle, chloro,
R^{3'} hydrogène, méthyle,
R⁴ méthyle, trifluorométhyle,
R⁵ chloro,
R⁶ hydrogène ou méthyle;
R alkyle en C₃-C₁₂ éventuellement substitué par un radical halogène, alcényle en C₃-C₁₂ éventuellement substitué par un halogène, alcynyle en C₃-C₆, alcoxy en C₂-C₁₂ éventuellement substitué par un halogène, alcényloxy en C₃-C₁₂ éventuellement substitué par un halogène, alcynyloxy en C₃-C₁₂, cycloalkyle en C₃-C₆ éventuellement substitué par un radical alkyle en C₁-C₄, cycloalcényle en C₄-C₆ éventuellement substitué par un radical alkyle en C₁-C₄, cycloalkyloxy en C₅-C₆ éventuellement substitué par un radical alkyle en C₁-C₄, cycloalcényloxy en C₅-C₆ éventuellement substitué par un radical alkyle en C₁-C₄,
à l'exception des composés I dans lesquels
a)
A = A2',
R = éthoxy éventuellement substitué par un halogène et
b)
A = A4',
R = cycloalkyle, cycloalcényle, cycloalkyloxy ou cycloalcényloxy, éventuellement substitués, et
c)
A = A4',
R = alkyle, alcoxy, alcényle, alcynyle, éventuellement substitués par un halogène.

6. Amides d'acides carboxyliques de formule générale I selon la revendication 1, dans lesquels les restes représentent les groupes suivants: 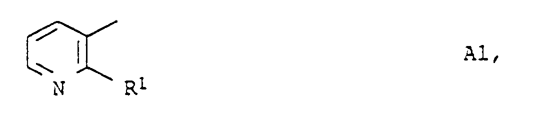
R¹ halogéno, méthyle, trifluorométhyle, méthoxy, méthylsulfinyle, méthylsulfonyle,
R alkyle en C₃-C₁₂ éventuellement substitué par un hélogène, alcényle en C₃-C₁₂ éventuellement substitué par un halogène, alcynyle en C₃-C₆, alcoxy en C₂-C₁₂ éventuellement substitué par un halogène, alcényloxy en C₃-C₁₂ éventuellement substitué par un halogène, alcynyloxy en C₃-C₁₂, cycloalkyle en C₃-C₆, cycloalcényle en C₄-C₆, cycloalkyloxy en C₅-C₆, cycloalcényloxy en C₅-C₆,
à l'exception des composés I dans lesquels
A = A1, qui porte un halogène en position 2,
R = isopropyle, et
A = A1, qui porte un halogène en position 2,
R = éthoxy éventuellement substitué par un halogène.

7. Amides d'acides carboxyliques de formule générale I selon la revendication 1, dans lesquels les restes représentent les groupes suivants:
A 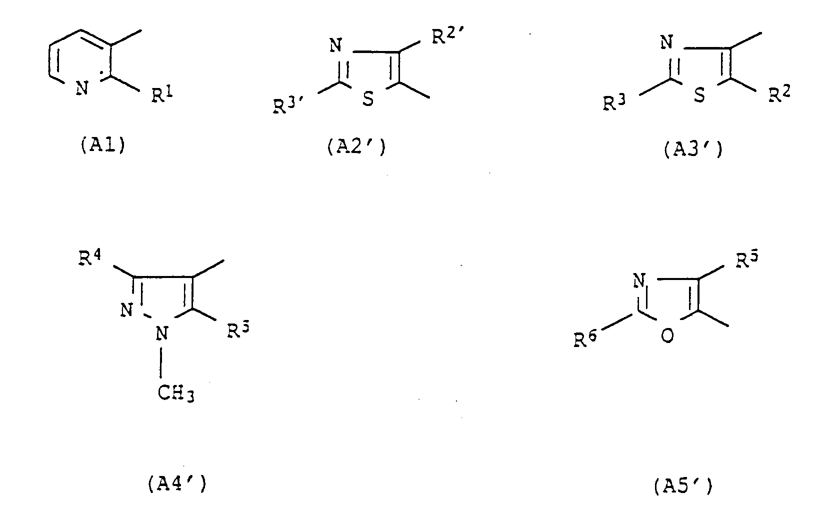
où les substituants R¹ à R⁶ représentent quant à eux les groupes:
R¹ trifluorométhyle, chloro,
R² méthyle, trifluorométhyle, chloro,
R^{2'} méthyle, trifluorométhyle,
R³ hydrogène, méthyle, chloro,
R^{3'} hydrogène, méthyle,
R⁴ méthyle, trifluorométhyle,
R⁵ chloro,
R⁶ hydrogène ou méthyle;
R phényle, qui peut être substitué une fois par un groupe alkyle en C₁-C₄, alcoxy en C₁-C₄, alkylthio en C₁-C₄, halogéno,
à l'exception des composés I dans lesquels
a)
A = A1, qui porte un halogène en position 2,
R = phényle non-substitué,
b)
A = A2',
R = phényle non-substitué et
c)
A = A4',
R = phényle non-substitué.

8. Agent botryticide, contenant une quantité à activité botryticide d'au moins un des composés I selon l'une quelconque des revendications 5 à 7 et un support solide ou liquide.
